# EUROPEAN PATENT SPECIFICATION

(11) **EP 3 826 762 B1**
(45) Date of publication and mention of the grant of the patent: **02.12.2026**
(21) Application number: 19748705.1
(22) Date of filing: 05.07.2019
(51) Int. Cl.: B01J 19/00, G06N 3/12, C12Q 1/6874

(54) **MASSIVELY PARALLEL ENZYMATIC SYNTHESIS OF NUCLEIC ACID STRANDS**
MASSIV PARALLELE ENZYMATISCHE SYNTHESE VON NUKLEINSÄURESTRÄNGEN
SYNTHÈSE ENZYMATIQUE MASSIVEMENT PARALLÈLE DE BRINS D'ACIDES NUCLÉIQUES

(30) Priority: 23.07.2018 EP 18306000
(43) Date of publication of application: 02.06.2021
(73) Proprietor: DNA Script, 94270 Le Kremlin-Bicêtre (FR)
(72) Inventor: HORGAN, Adrian, 75014 Paris (FR); GODRON, Xavier, 75014 Paris (FR); YBERT, Thomas, 75012 Paris (FR); NICHOL, Robert, 94270 LE KREMLIN-BICÊTRE (FR)
(74) Representative: Plasseraud IP
(86) International application number: PCT/EP2019/068183
(87) International publication number: WO 2020/020608

(56) References cited:
- WO-A1-2016/094512
- WO-A1-2017/176541
- WO-A1-2017/196783
- WO-A1-2020/178604
- WO-A2-2014/014991
- US-A1- 2004 238 369
- US-A1- 2007 034 513
- US-A1- 2018 201 968

## Description

The present invention relates to new process for massively parallel synthesis of nucleic acid strands.

Biology can store information with extraordinary density, stability, and efficiency with the potential to outperform electronic based long-term data storage by orders of magnitude. Nature can routinely store and extract more than 3 gigabytes of information in a human cell and coordinate hierarchical delivery of information to differentiate tissues and regulate metabolic processes. However, storage and access of this information is tightly coupled to the biological processes encoded in the data; therefore, its adaptation to digital data storage is severely limited by our current abilities to read and write DNA at comparable semiconductor digital scales. Recent advances in DNA sequencing have greatly increased our reading capability with throughputs exceeding terabases of DNA per sequencing run opening the possibility to read at high throughput, although even this throughput is insufficient to achieve comparable throughput to digital systems. DNA synthesis capacity lags behind sequencing capacity by at least two orders of magnitude in terms of throughput and cost. Nevertheless, demonstrations using existing DNA synthesis and sequencing technologies have demonstrated the power of this approach for data storage, e.g. Church et al, Science, 337(6102): 1628 (2012); Organick et al, Nature Biotechnology, 36(3): 242-248 (2018); and the like. The challenge is that the dense hierarchical data storage in nature requires breakthroughs in encoding algorithms, DNA synthesis, and DNA sequencing together to deliver a true molecular based data storage capability.

In particular, current approaches to DNA data storage treat reading and writing as separate processes, so that information is encoded in DNA in the course of synthesis, the synthesized and encoded DNA strands are separately stored, and information is retrieved by selective amplification and sequencing, e.g. Bornholt et al, IEEE Micro, 37(3): 98-104 (2017). The high information storage capacity of DNA could be exploited more advantageously if DNA storage devices were available that supported on the same substrate both the writing, or synthesis, of DNA and the reading, or sequencing, of DNA.

WO2017/196783 describes a method for inactivating or activating an enzyme, such as a polymerase, by altering the pH through the application of a voltage to an electrochemical cell. WO2020/178604 discloses a method for enzymatic synthesis of DNA.

### Summary of the Invention

The present invention is directed to methods of parallel template-free enzymatic synthesis of a plurality of nucleic acids; and more particularly, methods of parallel template-free enzymatic synthesis of nucleic acids with localized electrochemical de-protection steps of a 3'-O electrochemically labile protecting group which is the amino group. Localized electrochemical deprotection may be accomplished in a variety of ways including, for example, controlling of pH at a local reaction site to deprotect pH-sensitive bonds and/or controlling electrical potential. In some embodiments, electrochemical deprotection is implemented locally using an electrode array, where control of the electrochemical properties of each reaction site is determined by one or more associated electrodes. In some embodiments, the invention is directed to parallel synthesis of polynucleotides on a substrate for storing information and to sequencing the same polynucleotides on the same substrate for retrieving information. In some embodiments, such sequencing is carried out using a sequencing-by-synthesis methodology that employs deoxynucleoside triphosphates (dNTPs) which may have a label removable by localized electrochemical changes and/or a 3'-O-blocking group removable by localized electrochemical changes.

It is a further purpose of the present invention to integrate multiple advanced technologies required to deliver a molecular data storage system that will be engineered, tested, and optimized with best industrial practices. This requires advanced new DNA synthesis technologies, new DNA sequencing methods, microscale manufacturing, microfluidics, and robust encoding. To this end, the present invention proposes to leverage the molecular precision and diversity of biological encoding systems by using enzymatic nucleic acid synthesis and associated biological machinery, to use microsystems and microfluidics to gain precise control over biological chemistry enabling highly parallelized synthesis, readout, and storage, and/or to use rapid design-build-test iteration, process control toolbox including design of experiments to deliver fully integrated systems rather than individual technologies.

The process of the present invention provides technical solution for the storage, retrieval and operating system. More particularly, the storage part is addressed by combining the potential of novel enzymatic DNA synthesis technologies and existing and proven highly parallelized automation approached. The retrieval part is addressed by leveraging existing "sequencing by synthesis" (SBS) technologies optimized to work with specific DNA structure, barcoding and density, in combination with the optimization of data structure. The operating system part presents a scheme for optimization of data density.

In some embodiments, the plurality of polynucleotides produced by the method of the invention may be combined to form larger fragments, such as genes for use in synthetic biology.

In some embodiments, template-free enzymatic synthesis methods of the invention may be used to append further information to pre-existing polynucleotides carrying encoded information. Such added information may correct pre-existing information, e.g. as in correcting or up-dating an address, or added information may simply negate or void the pre-existing information in some sense.

In one such embodiment, polynucleotides containing pre-existing information may be seeded on reaction sites and amplified under kinetic exclusion conditions or by template walking, e.g. Ma et al, Proc. Natl. Acad. Sci., 110(35): 14320-14323 (2013); U.S. patents 9476080; 8895249; and the like. New information is added to the cloned polynucleotides by enzymatically coupling a predetermined sequence of nucleotides onto the ends of the cloned polynucleotides. The predetermined sequence of nucleotides may include the new information in a coded format. In some embodiments, prior to adding new information the cloned polynucleotides are sequenced in part or entirely, so that the new information added at a particular reaction site may depend on the information content extracted from the cloned polynucleotide at the particular site by the initial sequencing. In some embodiments, the cloned polynucleotides that have been augmented with new information may be cleaved from the reaction sites for storage or for further processing steps.

In some embodiments, polynucleotides may be synthesized using modified nucleotides that are more resistant to degradation than natural nucleotides, thereby increasing storage life and information integrity. In some embodiments, phosphorothioate, 2'-fluoro, or 2'-O-Me nucleotide monomers are substituted for natural nucleotide monomers, either entirely or as a proportion of the nucleotides in the synthesized strands, for example, to reduce enzymatic degradation. In some embodiments, completed polynucleotides are "capped" by phosphorylating their 3'-ends, for example, to reduce the likelihood of exonuclease digestion. In some embodiments, deamination may be reduced by employing 3'-O-protected dNTPs that also have base-protection groups on exocyclic amines, e.g. N-benzyl-dATP, N-benzyl-dCTP, N-isobutyl-dGTP, or the like, e.g. Beaucage and Iyer, Tetrahedron, 48(12): 2223-2311 (1992)(especially Table 3); Narang, Chapter 1, in Synthesis and Applications of DNA and RNA (Academic Press, Orlando, 1987); Srivastava et al, International patent publication WO2010/134992; and the like.

In some embodiments, polynucleotides with encoded information may be stored or maintained in double stranded form, which, for example, is more resistant to depurination. In other embodiments, coding schemes are selected that maximize the use dA's over other monomers and minimize the use of dT's in order to maximize resistance to depurination. In a particular such embodiment, information is encoded using only dAs, dCs and dGs. In some embodiments, methods of the invention comprising steps of synthesizing (writing) polynucleotides and sequencing (reading) polynucleotides include a step of proof-reading a newly synthesized polynucleotide by a sequencing-by-synthesis method that results in a double stranded product for storage. For later retrieval of information encoded in the polynucleotide, the sequencing strand may be melted off and subjected to a re-sequencing step.

In some embodiments, information-containing polynucleotides are stored in a carrier solution, such as, a readily available natural DNAs, such as salmon sperm DNA, a polycation, such as spermidine, polyvinylpyrrolidones, polymethylmethacrylates, or the like.

In some embodiments, methods of the invention for synthesizing a plurality of polynucleotides in parallel comprise the following steps: (a) providing a spatially addressable array of reaction sites, wherein each reaction site is operationally associated with at least one working electrode and has disposed thereon initiators attached by their 5'-ends and having a 3'-O-electrochemically labile protecting group; (b) performing for each kind of nucleotide a cycle of (i) deprotecting initiators or elongated fragments at electrodes at predetermined addresses by generating a voltage difference between each of the electrodes at the predetermined addresses and a reference electrode so that the electrochemically labile protecting group is cleaved, thereby generating free 3'-hydroxyls on the initiators or elongated fragments at the electrodes of the predetermined addresses, (ii) contacting under elongation conditions the electrodes at the predetermined addresses with a 3'-O-electrochemically labile-protected nucleoside triphosphate and a template-independent DNA polymerase so that the initiators or elongated fragments at the predetermined addresses are elongated by the incorporation of a 3'-electrochemically labile-protected nucleoside triphosphate to form 3'-O-electrochemically labile-protected elongated fragments; and (c) repeating step (b) until the array of polynucleotides of predetermined sequences is completed, wherein said electrochemically labile protecting group is pH sensitive and wherein said voltage difference between said electrodes at said predetermined addresses and a reference electrode activates an electroactive agent at said predetermined addresses which changes the pH at said predetermined addresses, thereby cleaving said electrochemically labile protecting group, wherein said electrochemically labile protecting group is an amino group and wherein said spatially addressable array of reaction sites is integrated in a semiconductor device, in particular a Complementary Metal Oxide Semiconductor (CMOS) device.

In some embodiments, the invention is directed to a method of synthesizing a plurality of polynucleotides having predetermined sequences, the method comprising the steps of:
(a) providing a spatially addressable array of reaction sites, wherein each reaction site is operationally associated with at least one working electrode and has disposed thereon initiators attached by their 5'-ends and having a 3'-O-electrochemically labile protecting group;
(b) performing for each kind of nucleotide a cycle of (i) deprotecting initiators or elongated fragments at electrodes at predetermined addresses by generating a voltage difference between each of the electrodes at the predetermined addresses and a reference electrode so that the electrochemically labile protecting group is cleaved, thereby generating free 3'-hydroxyls on the initiators or elongated fragments at the electrodes of the predetermined addresses, (ii) contacting under elongation conditions the electrodes at the predetermined addresses with a 3'-O-electrochemically labile-protected nucleoside triphosphate and a template-independent DNA polymerase so that the initiators or elongated fragments at the predetermined addresses are elongated by the incorporation of a 3'-electrochemically labile-protected nucleoside triphosphate to form 3'-O-electrochemically labile-protected elongated fragments; and
(c) repeating step (b) until the array of polynucleotides of predetermined sequences is completed,
wherein said electrochemically labile protecting group is an amino group and wherein said voltage difference between said electrodes at said predetermined addresses and a reference electrode activates an electroactive agent at said predetermined addresses which changes the pH at said predetermined addresses, thereby cleaving said electrochemically labile protecting group, wherein said electroactive agent is selected from hydroquinone, benzoquinone, quinone, and derivatives thereof.

In some embodiments, the invention is directed to a method of template-free enzymatic synthesis of a polynucleotide with proofreading. Such method may be implemented with the following steps: a) providing an initiator at a reaction site operationally associated with at least one working electrode, wherein the initiator has a free 3-O-hydroxyl; b) repeating cycles of (i) contacting under elongation conditions the initiator or an elongated fragment thereof having free 3'-O-hydroxyls with a 3'-O-electrochemically labile-protected nucleoside triphosphate and a template-independent DNA polymerase so that the initiator or elongated fragment thereof is elongated by the incorporation of a 3'-electrochemically labile-protected nucleoside triphosphate to form 3'-O-electrochemically labile-protected elongated fragment; and (ii) deprotecting the elongated fragment of step (i) to form elongated fragment having a free 3'-hydroxyl, until the polynucleotide is complete and a sequencing primer binding site is appended to its 3' end; and c) annealing a sequencing primer to the sequencing primer binding site and sequencing the polynucleotide, wherein said 3'-O-electrochemically labile protecting group is an amino group and wherein said voltage difference between said electrodes at said predetermined addresses activates an electroactive agent at said predetermined addresses which changes the pH at said predetermined addresses, thereby cleaving said 3'-O-electrochemically labile protecting group, wherein said electrochemically labile protecting group is an amino group.

In some embodiments, the invention is directed to a method of storing and retrieving information on and from an array of polynucleotides. Such method may be implemented by the following steps: (a) providing a spatially addressable array of reaction sites, wherein each reaction site is operationally associated with at least one working electrode and has disposed thereon initiators attached by their 5'-ends and having a 3'-O-electrochemically labile protecting group; (b) performing for each kind of nucleotide a cycle of (i) deprotecting initiators or elongated fragments at electrodes at predetermined addresses by generating a predetermined voltage difference between each of the electrodes at the predetermined addresses and a reference electrode so that the electrochemically labile protecting group is cleaved, thereby generating free 3'-hydroxyls on the initiators or elongated fragments at the electrodes at the predetermined addresses, (ii) contacting under elongation conditions the electrodes at the predetermined addresses with a 3'-O-electrochemically labile-protected nucleoside triphosphate and a template-independent DNA polymerase so that the initiators or elongated fragments at the predetermined addresses are elongated by the incorporation of a 3'-electrochemically labile-protected nucleoside triphosphate to form 3'-O-electrochemically labile-protected elongated fragments; (c) repeating step (b) until the array of polynucleotides of predetermined sequences is completed, wherein each of the completed polynucleotides comprises in a 5' to 3' direction an information encoding region and a sequencing primer binding site at its 3' end; and (d) retrieving information from the information encoding region by annealing a sequencing primer to the sequencing primer binding site and sequencing by synthesis the completed polynucleotides at one or more reaction sites, wherein said electrochemically labile protecting group is an amino group.

### Brief Description of the Drawings

Fig. 1A contains a schematic representation of an enzymatic synthesis cycle wherein a 3'-O-protected dNTP is added to a nucleic acid strand followed by de-protection. Fig. 1B illustrates the steps of one embodiment of the invention for parallel template-free enzymatic synthesis of a plurality of polynucleotides on an addressable electrode array.
Fig. 2 illustrates a design of an oligonucleotide for data storage (A) and a design of an oligonucleotide for data storage employing an intermediary primer (B).
Fig. 3 compares binary encoding (prior art), standard DNA quaternary encoding (prior art), presence/absence combinatorial encoding (mixes of bases where the data is encoded in the presence of absence of a nucleotide at each cycle) according to the invention, and 25% combinatorial encoding (mixes of bases where the data is encoded in the % of each base at each cycle - with 5 levels of % possible for each base: 0%, 25%, 75% and 100%) according to the invention.
Fig. 4 presents an example of encoding using the absence/presence combinatorial scheme of the invention, as presented in FIGURE 3 on one well of a DNA array. The data is encoded in base 14 (X1, X2, ..., X14). The code X1 X2 X10 X6 X11 X8 represented here is stored on 6nt long DNA strands. It encodes more than 22bits of data. A 12 nucleotide DNA fragment would have been needed to store the same amount of data using quaternary encoding.
Fig. 5 illustrate an example of pseudo 3D DNA data storage. The microarray grid is in 2D and the DNA sequence (or mix of sequence in the combinatorial scheme) is the 3^{rd} dimension.
Fig. 6A is an embodiment of a potentiostat electronic circuit for controlling electrochemical conditions at an electrode.
Fig. 6B illustrates diagrammatically an embodiment of an apparatus for synthesizing polynucleotides on an electrode array in which reagents are flowed across the array in sequence.
Fig. 6C illustrates diagrammatically an embodiment of an apparatus for synthesizing polynucleotides on an electrode array which uses droplet delivery of reagents to reaction sites of the electrodes.
Figs. 7A-7D illustrates data storage techniques possible with template-free enzymatic synthesis.
Fig. 8 shows an electropherogram of extension products from a reaction in which a mutant TdT that cannot incorporate 3'-O-azidomethyl-protected dNTPs successfully extended primers with 3'-O-hydroxyl dNTP which, in turn, were products from an electrochemically deprotection reaction, thereby demonstrating the possibility of electrochemically deprotecting 3'-O-azidomethyl-nucleotides.
Fig. 9 illustrates efficiency of deprotecting 3'-O-NH2-nucleotides (that is, converting 3'-O-NH2 to 3'-OH) versus pH for several incubation times.

### DETAILED DESCRIPTION OF THE INVENTION

While the invention is amenable to various modifications and alternative forms, specifics thereof have been shown by way of example in the drawings and will be described in detail. It should be understood, however, that the intention is not to limit the invention to the particular embodiments described. The intention is to cover all modifications, equivalents, and alternatives falling within the spirit and scope of the invention. For example, the microelectronics portion of the apparatus and array is implemented in CMOS technology for purposes of illustration. It should be appreciated, however, that the disclosure is not intended to be limiting in this respect, as other semiconductor-based technologies may be utilized to implement various aspects of the microelectronics portion of the systems discussed herein. Guidance for making arrays of the invention is found in many available references and treatises on integrated circuit design and manufacturing and micromachining, including, but not limited to, Allen et al, CMOS Analog Circuit Design (Oxford University Press, 2nd Edition, 2002); Levinson, Principles of Lithography, Second Edition (SPIE Press, 2005); Doering and Nishi, Editors, Handbook of Semiconductor Manufacturing Technology, Second Edition (CRC Press, 2007); Baker, CMOS Circuit Design, Layout, and Simulation (IEEE Press, Wiley-Interscience, 2008); Veendrick, Deep-Submicron CMOS ICs (Kluwer-Deventer, 1998); Cao, Nanostructures & Nanomaterials (Imperial College Press, 2004); and the like, which relevant parts are hereby incorporated by reference. Likewise, guidance for carrying out electrochemical measurements of the invention is found in many available references and treatises on the subject, including, but not limited to, Sawyer et al, Electrochemistry for Chemists, 2nd edition (Wiley Interscience, 1995); Bard and Faulkner, Electrochemical Methods: Fundamentals and Applications, 2nd edition (Wiley, 2000); and the like, which relevant parts are hereby incorporated by reference.

In one aspect, the present invention provides a new process allowing massively parallel enzymatic synthesis of polynucleotides. As mentioned above, in one aspect, the method employs electrochemically labile protecting groups for facile parallel synthesis on large-scale electrode arrays. In one application of this process polynucleotides are used to store data which can be later retrieved from the same synthesis support by a DNA sequencing operation, for example, using a sequencing by synthesis technique, particularly one employing electrochemically labile blocking groups and/or labels.

### A. Background of Enzymatic DNA Synthesis.

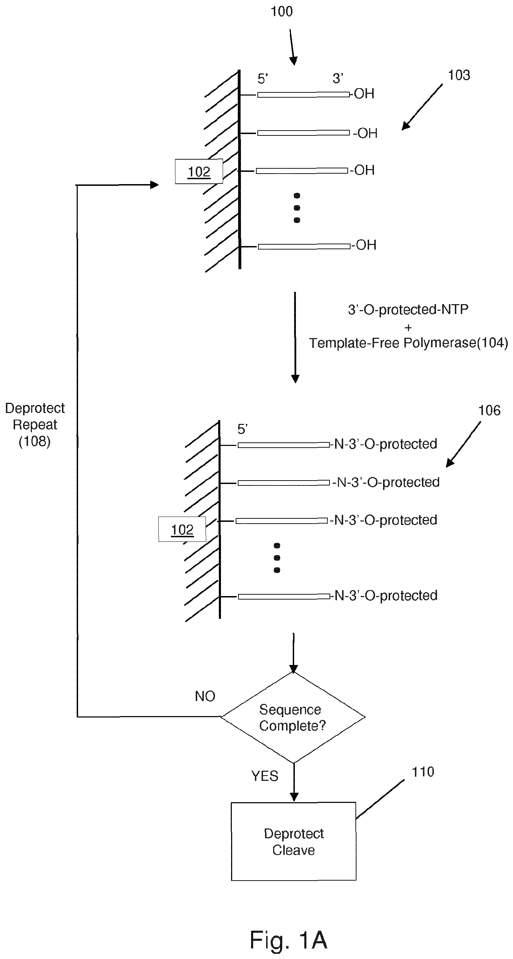

Recently an enzymatic synthesis process to produce very long DNA or RNA strands with the best purity has been developed (WO2015/159023). A cycle of the enzymatic synthesis process, leading to the addition of a nucleotide to a nucleic acid strand, comprises two successive steps, corresponding to an elongation step and a deprotecting step respectively (Fig. 1A). In summary, during the elongation step, the polymerase adds a nucleotide comprising a protecting group to a nucleic acid strand. Then the protection group is removed from this newly added nucleotide, to be able to perform additional cycles. This new enzymatic synthesis technology is bringing dramatic improvement to nucleic acid synthesis, as compared to chemical synthesis. The key advantages for data storage are described in the table below:

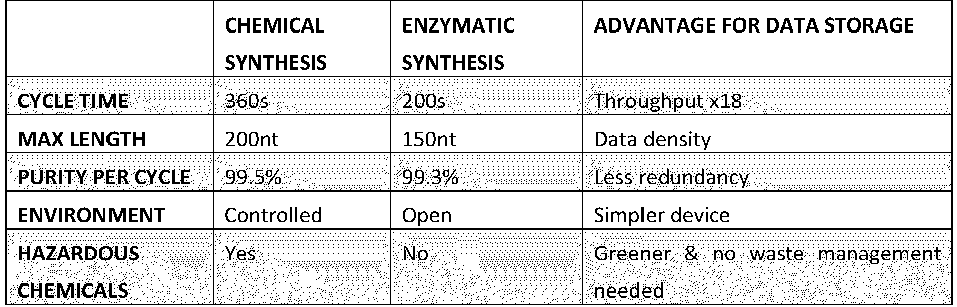

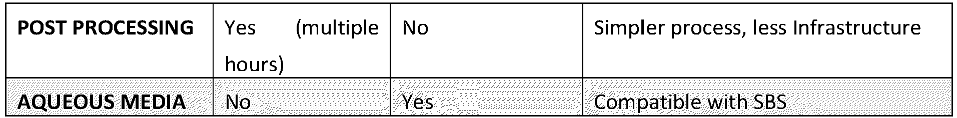

Generally, methods of template-free enzymatic DNA synthesis comprises repeated cycles of steps, such as are illustrated in Fig. 1A, in which a predetermined nucleotide is coupled to an initiator or growing chain in each cycle. The general elements of template-free enzymatic synthesis is described in the following references: Ybert et al, International patent publication WO/2015/159023; Ybert et al, International patent publication WO/2017/216472; Hyman, U.S. patent 5436143; Hiatt et al, U.S. patent 5763594; Jensen et al, Biochemistry, 57: 1821-1832 (2018); Mathews et al, Organic & Biomolecular Chemistry, DOI: 0.1039/c6ob01371f (2016); Schmitz et al, Organic Lett., 1(11): 1729-1731 (1999).

Initiator polynucleotides (100) are provided, for example, attached to solid support (102), which have free 3'-hydroxyl groups (103). To the initiator polynucleotides (100) (or elongated initiator polynucleotides in subsequent cycles) are added a 3'-O-protected-dNTP and a template-free polymerase, such as a TdT or variant thereof (e.g. Ybert et al, WO/2017/216472) under conditions (104) effective for the enzymatic incorporation of the 3'-O-protected-dNTP onto the 3' end of the initiator polynucleotides (100) (or elongated initiator polynucleotides). This reaction produces elongated initiator polynucleotides whose 3'-hydroxyls are protected (106). If the elongated initiator polynucleotide contains a competed sequence, then the 3'-O-protection group may be removed, or deprotected, and the desired sequence may be cleaved from the original initiator polynucleotide. Such cleavage may be carried out using any of a variety of single strand cleavage techniques, for example, by inserting a cleavable nucleotide at a predetermined location within the original initiator polynucleotide. An exemplary cleavable nucleotide may be a uracil nucleotide which is cleaved by uracil DNA glycosylase. If the elongated initiator polynucleotide does not contain a completed sequence, then the 3'-O-protection groups are removed to expose free 3'-hydroxyls (103) and the elongated initiator polynucleotides are subjected to another cycle of nucleotide addition and deprotection. In accordance with the invention, 3'-O- protection groups are electrochemically labile groups. That is, deprotection or cleavage of the protection group is accomplished by changing the electrochemical conditions in the vicinity of the protection group which result in cleavage. Such changes in electrochemical conditions may be brought about by changing or applying a physical quantity, such as a voltage difference or light to activate auxiliary species which, in turn, cause changes in the electrochemical conditions at the site of the protection group, such as an increase or decrease in pH. In some embodiments, electrochemically labile groups include, for example, pH-sensitive protection groups that are cleaved whenever the pH is changed to a predetermined value. Electrochemically labile groups also include protecting groups which are cleaved directly whenever reducing or oxidizing conditions are changed, for example, by increasing or decreasing a voltage difference at the site of the protection group. According to the invention, the electrochemically labile protecting group is an amino group.

As used herein, an "initiator" (or equivalent terms, such as, "initiating fragment", "initiator nucleic acid", "initiator oligonucleotide", or the like) usually refers to a short oligonucleotide sequence with a free 3'-end, which can be further elongated by a template-free polymerase, such as TdT. In one embodiment, the initiating fragment is a DNA initiating fragment. In an alternative embodiment, the initiating fragment is an RNA initiating fragment. In some embodiments, an initiating fragment possesses between 3 and 100 nucleotides, in particular between 3 and 20 nucleotides. In some embodiments, the initiating fragment is single-stranded. In alternative embodiments, the initiating fragment is double-stranded. In some embodiments, an initiator may comprise a non-nucleic acid compound having a free hydroxyl to which a TdT may couple a 3'-O-protected dNTP, e.g. Baiga, U.S. patent publications US2019/0078065 and US2019/0078126.

Returning to Fig. 1A, in some embodiments, an ordered sequence of nucleotides are coupled to an initiator nucleic acid using a template-free polymerase, such as TdT, in the presence of 3'-O-protected dNTPs in each synthesis step. In some embodiments, the method of synthesizing an oligonucleotide comprises the steps of (a) providing an initiator having a free 3'-hydroxyl; (b) reacting under extension conditions the initiator or an extension intermediate having a free 3'-hydroxyl with a template-free polymerase in the presence of a 3'-O-protected nucleoside triphosphate to produce a 3'-O-protected extension intermediate; (c) deprotecting the extension intermediate to produce an extension intermediate with a free 3'-hydroxyl; and (d) repeating steps (b) and (c) until the polynucleotide is synthesized. (The terms "extension intermediate," "extension product" and "elongation fragment" are used interchangeably). In some embodiments, an initiator is provided as an oligonucleotide attached to a solid support, e.g. by its 5' end. The above method may also include washing steps after the reaction, or extension, step, as well as after the de-protecting step. For example, the step of reacting may include a sub-step of removing unincorporated nucleoside triphosphates, e.g. by washing, after a predetermined incubation period, or reaction time. Such predetermined incubation periods or reaction times may be a few seconds, e.g. 30 sec, to several minutes, e.g. 30 min.

The 3'-O-blocked dNTPs employed in the invention may be purchased from commercial vendors or synthesized using published techniques, e.g. U.S. patent 7057026; Guo et al, Proc. Natl. Acad. Sci., 105(27): 9145-9150 (2008); Benner, U.S. patent 7544794.

The above method may also include capping step(s) as well as washing steps after the reacting, or extending, step, as well as after the deprotecting step. As mentioned above, in some embodiments, capping steps may be included in which non-extended free 3'-hydroxyls are reacted with compounds that prevents any further extensions of the capped strand. In some embodiments, such compound may be a dideoxynucleoside triphosphate. In other embodiments, non-extended strands with free 3'-hydroxyls may be degraded by treating them with a 3'-exonuclease activity, e.g. Exo I. For example, see Hyman, U.S. patent 5436143. Likewise, in some embodiments, strands that fail to be deblocked may be treated to either remove the strand or render it inert to further extensions.

In some embodiments, reaction conditions for an extension or elongation step may comprising the following: 2.0 µM purified TdT; 125-600 µM 3'-O-protected dNTP (e.g. 3'-O-NH₂-protected dNTP); about 10 to about 500 mM potassium cacodylate buffer (pH between 6.5 and 7.5) and from about 0.01 to about 10 mM of a divalent cation (e.g. CoC1₂ or MnC1₂), where the elongation reaction may be carried out in a 50 µL reaction volume, at a temperature within the range RT to 45°C, for 3 minutes.

Enzymatic nucleic acid synthesis enables to synthesize longer and purer DNA fragments, faster than chemistry. The cycle time factor is particularly interesting for data storage as it enables to increase the throughput 15 to 20-fold. Performing the synthesis in aqueous media also makes it greener (no organic solvents used during synthesis), simplifies instrumentation (no need to control the environment) and eliminates the need for chemical waste management facilities.

The present invention now proposes to improve this enzymatic synthesis process to allow massively parallel synthesis. In a first aspect, the present invention provides a process compatible with pH controlled deprotection.

### B. Approaches to Parallel Enzymatic Polynucleotide Synthesis.

In one aspect, the invention provides methods and apparatus for highly parallel template-free enzymatic synthesis of a plurality of different polynucleotides each having a predetermined sequence of nucleotides. In some embodiments, parallel synthesis is implemented by providing a support having discrete, non-overlapping, addressable sites where separate polynucleotides are synthesized and a means for controlling electrochemical conditions at each site independently of the other sites is provided. In some embodiments, such parallel synthesis support is a planar support having a regular pattern of addressable sites, such as, a rectilinear pattern of sites, or a hexagonal pattern of sites. In some embodiments, each site of a planar support is associated with one or more electrodes whose electrical characteristics may be controlled in an addressable manor independent of other electrodes of the planar support. In some embodiments, such planar supports have a plurality of sites comprising at least 256 sites, at least 512 sites, at least 1024 sites, at least 5000 sites, at least 10,000 sites, at least 25,000 sites, or at least 100,000 sites and as many as 10,000,000 sites. In some embodiments, such planar supports have a plurality of sites greater than 1000, or 10,000, or 25,000, or 50,000, or 100,000, or 500,000, and up to 1,000,000 sites or up to 10,000,000 sites. In some embodiments, the sites of such planar supports are disposed in a regular array and each site is associated with at least one electrode integrated with the planar support. In some embodiments, the discrete site at which synthesis and/or sequencing take place each has an area in the range of from .25 µm² to 1000 µm², or from 1 µm² to 1000 µm², or from 10 µm² to 1000 µm², or from 100 µm² to 1000 µm². In some embodiments, the amount of polynucleotides synthesized at each site is at least 10⁻⁶ fmol, or at least 10⁻³ fmol, or at least 1 fmol, or at least 1 pmol, or the amount of polynucleotide synthesized at each site is in the range of from 10⁻⁶ fmol to 1 fmol, or from 10⁻³ fmol to 1fmol, or from 1 fmol to 1 pmol, or from 10⁻⁶ pmol to 10 pmol, or from 10⁻⁶ pmol to 1 pmol. In some embodiments, the number of polynucleotides synthesized at each site is in the range of from 1000 molecules to 10⁶ molecules, or from 1000 molecules to 10⁹ molecules, or from 1000 molecules to 10¹² molecules.

In some embodiments, enzymatically synthesized polynucleotides at each reaction site have lengths in the range of from 50 to 500 nucleotides; in other embodiments, such polynucleotides have lengths in the range of from 50 to 1000 nucleotides.

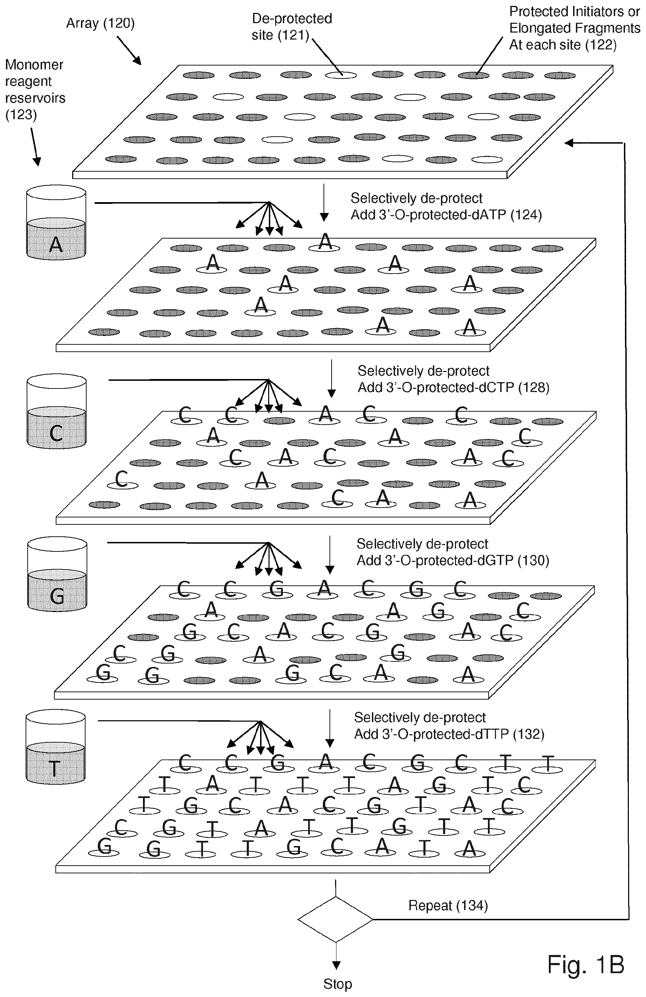

Fig. 1B illustrates the steps for one embodiment of parallel synthesis of a plurality of polynucleotides on discrete sites addressable either for specific photo-illumination or for electrode activation. In some embodiments, the array is an addressable electrode array in which individual electrodes may be controlled to generate a predetermined voltage difference between any given working electrode of the array and a counter electrode. Array (120) is provided such that each site (122) has an initiator or an elongated fragment with a protected 3'-hydroxyl (represented as the dark disks). To initiate a synthesis cycle, the 3'-hydroxyls of initiators or elongated fragments of selected sites (the ones corresponding to polynucleotides for which the next monomer is A) are deprotected (121) (represented as open disks) using a deprotection method that can be restricted to the location of the selected sites. As described more fully below, in some embodiments, such localized deprotection may be effected by localized photoreactions or by localized changes in voltage differences using site-specific electrodes. To the selectively deprotected sites is added a reagent comprising 3'-O-protected-dATPs (124) and a template-free polymerase, such as a TdT, is delivered to the deprotected sites. As described briefly below, the synthesis reagents may be delivered in a variety of ways, such as, by simple bulk flow over the entire array, droplets delivered by an inkjet device to individual sites, or the like. After a predetermined time for the coupling reaction to advance to completion or to a suitable extent, the array is washed and the next group of polynucleotides (those for which C is the next monomer) at selected sites have their 3'-hydroxyls deprotected. To the selectively deprotected sites is added a reagent comprising 3'-O-protected-dCTPs (128) and a template-free polymerase, such as a TdT, is delivered to the deprotected sites. Similar steps are performed for dGTPs (130) and dTTPs (132), until the cycle is completed. The cycles are repeated (134) until the polynucleotides are completed.

### Photo-Induced Deprotection.

In some embodiments, the process of the invention uses photo induced deprotection with a photogenerated acid to locally deprotect, e.g. Gao et al, U.S. patents 6426184, 7491680 and 7838466. Advantageously, the oligonucleotides are synthesized in a flow cell, very similar to those used for Sequencing by Synthesis (SBS) today. SBS uses modified dNTPs containing a terminator which blocks further polymerization. So only a single base can be added by a polymerase enzyme to each growing DNA or RNA copy strand. The sequencing reaction is conducted simultaneously on a very large number of different template molecules spread out on a solid surface. Following the addition of the four dNTPs to the templates, the terminators are removed. This chemistry is called "reversible terminators". Finally, another four cycles of dNTP additions are initiated. Since single bases are added to all templates in a uniform fashion, the sequencing process produces a set of DNA/RNA sequence reads of uniform length. Advantageously, the DNA/RNA sample is prepared into a "sequencing library" by the fragmentation into pieces each around 200 bases long. Custom adapters are added to each end and the library is flowed across a solid surface (the "flow cell") and the template fragments bind to this surface. Following this, a solid phase "bridge amplification" PCR process (cluster generation) creates approximately one million copies of each template in tight physical clusters on the flowcell surface.

In some embodiments of the present invention, the chip can be directly read after synthesis by SBS to ensure successful encoding. In some embodiments, the chips can be approximately 50cm². The chip has a grid of microwells at a 30µm, 5µm, or even 1µm. With a 5µm pitch, the total number of wells is then 200 million, with 1µm pitch, the chip has 5 billion microwells. The oligonucleotides can be grafted directly on the bottom of the wells or on beads which would be filled in the chip in such a way that there is one and only one bead per chip. Deprotection may be effected by controlled by photo-generated acid in selected wells by using a Digital Micromirror Device (DMD).

In some embodiments, the oligonucleotides synthesized will be up to 400 nucleotides in length, as this is the maximum length easily readable by Sequencing By Synthesis using dual paired ends reads. Increasing the length of oligos above chemistry 200nt enables higher data density on the chip as data density as indexing will take a lower percentage of the oligonucleotide. Alternatively, and if synthesis purity enables it, it could be possible to increase the length synthesized and add intermediate primers every 200 nucleotides to ease sequencing to sequence sequentially the oligonucleotides.

One Flow cell with 5µm distance between wells enables the following:
- 200 million oligonucleotides of 400nt in parallel
- 7h to print the whole chip (80 000 000 000nt)
- 1.5 bit per cycle in encoding system (see below)
- 15GB per flow cell
With a 1µm pitch between wells (5 billion oligonucleotides in parallel), the amount of data stored is 375GB, performing this run 3 times enables to synthesize more than 1TB in one instrument.

The flow cell is preferably transparent for allowing UV deprotection and sequencing. The number of DMDs needed can be advantageously of at least 50. Preferably, the number of DMDs is more than 50, to increase number of wells and use confocal lenses in order to reduce pitch to 1µm and increase density.

One challenge in the generation of local pH changes is the diffusion of H3O+ ions. To prevent contamination, pH should be as stable as possible in the wells that are not illuminated and in the illuminated well during deprotection. Advantageously, during deprotection, the pH in the illuminated well is between 4,5 and 5,8, while the pH is maintained around 6 in non-illuminated wells.

To further increase the quantity of data stored, it is possible to develop photolabile nucleotides that are deprotected very quickly (up to about 1sec deprotection). Since the enzymatic technology is very robust and can be used in open environment, it is also possible to synthesis DNA/RNA on tapes instead of flow cells.

### Electrochemical deprotection.

Alternatively or in addition to local photochemical generation of pH changes, controlled changes in electrical potential at an electrode can be used to directly or indirectly cleave electrochemically labile groups. For example, pH-sensitive protection groups may be indirectly cleaved using voltage changes by employing an electroactive compound whose redox state may be changed by controlling local voltage differences, thereby liberating electrons which affect local pH, e.g. Southern, U.S. patent 5667667; Egeland and Southern, U.S. patent publication US2004/0238369; Egeland et al, Nucleic Acids Research, 33(14): e125 (2005); Maurer et al, U.S. patent 9267213; Fomina et al, LabChip, 16: 2236-2244 (2016), Moreover, chips enabling electrochemical deprotection on a large scale can be used, for example, by employing electrode arrays fabricated using CMOS chip technology or other semiconductor technology. Advantages of this technology include:
- Pitch reduction to permit massively large-scale synthesis
- Stackability of chips
- Synthesis and storage on 1µm beads
- Sequencing using semiconductor sequencing platforms, such as Ion Torrent
CMOS chips provide control to every synthesis sites by generating current or light to individually accessible sites. The current or light induced can be couple with respectively electrochemistry or photochemistry to generate locally protons in order to decrease the pH. In some embodiments, both photochemically generated acid and electrochemically generated acids is used to deprotect 3'-hydroxyls in the synthesis and/or sequencing process.

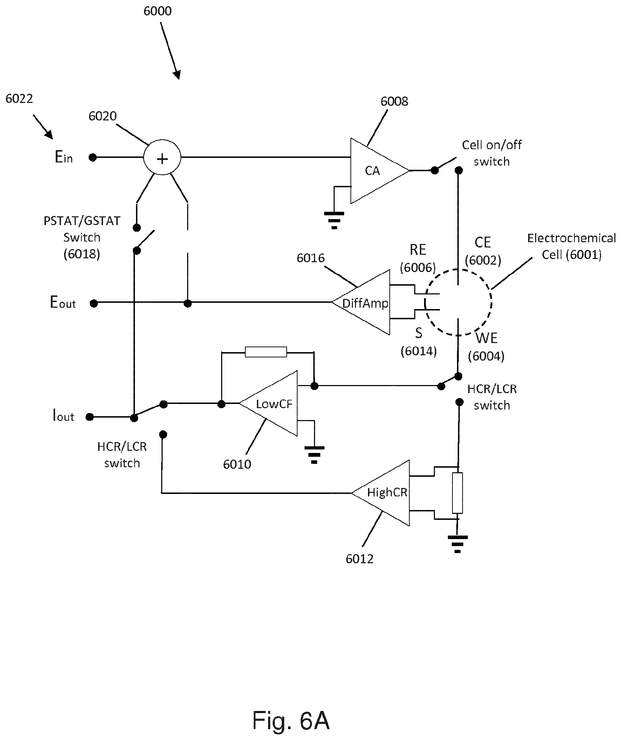

In some embodiments, each site on an electrode array may be configured as a potentiostat and/or galvanostat electrochemical cell (6001) as described by Levine et al (cited above) or Metrohm application note EC08. In potentiostatic mode, a potentiostat/galvanostat (PGSTAT) circuit (6000) as illustrated in Fig. 6A will accurately control the potential of Counter Electrode (CE) (6002) against the Working Electrode (WE)(6004) so that the potential difference between the working electrode (WE)(6004) and the Reference Electrode (RE)(6006) is well defined, and correspond to the value specified by the user. In galvanostatic mode, the current flow between the WE (6004) and the CE (6002) is controlled. The potential difference between the RE (6006) and WE (6004) and the current flowing between the CE (6002) and WE (6004) are continuously monitored. By using a PGSTAT, the value specified by the user (i.e. applied potential or current) is accurately controlled, anytime during the measurement by using a negative feedback mechanism.

As can be seen from the diagram, the CE (6002) is connected to the output of an electronic block referred to as a Control Amplifier (CA)(6008). The control amplifier forces current to flow through the cell. The value of the current is measured using a Current Follower (LowCF) (6010) or a Shunt (HighCR) (6012), for low and high currents, respectively. The potential difference is measured always between the RE (6006) and S (6014) with a Differential Amplifier (Diffamp)(6016). Depending on the mode the instrument is used (potentiostatic or galvanostatic) the PSTAT/GSTAT switch (6018) is set accordingly. The signal is then fed into the Summation Point (+) (6020) which, together with the waveform set by a digital-to-analog converter (Ein) (6022) will be used as an input for the control amplifier.

A counter electrode (also known as auxiliary electrode), is an electrode which is used to close the current circuit in the electrochemical cell. It is usually made of an inert material (e.g. Pt, Au, graphite, glassy carbon) and usually it does not participate in the electrochemical reaction. Because the current is flowing between the WE (6004) and the CE (6002), the total surface area of the CE (source/sink of electrons) is typically larger than the area of the WE so that it will not be a limiting factor in the kinetics of the electrochemical process.

A reference electrode is an electrode which has a stable and well-known electrode potential and it is used as a point of reference in the electrochemical cell for the potential control and measurement. The high stability of the reference electrode potential is usually reached by employing a redox system with constant (buffered or saturated) concentrations of each participants of the redox reaction. Moreover, the current flow through the reference electrode is kept close to zero (ideally, zero) which is achieved by using the CE to close the current circuit in the cell together with a very high input impedance on the electrometer (> 100 GOhm).

The working electrode is the electrode in an electrochemical system on which the reaction of interest is occurring. Common working electrodes can be made of inert materials such as Au, Ag, Pt, glassy carbon (GC) and Hg drop and film electrodes etc. Working electrode (6004) may comprise a coating for attaching molecules, such as initiators for template-free enzymatic polynucleotide synthesis.

Two electrode setup. In a two-electrode cell setup, CE (6002) and RE (6006) are shorted on one of the electrodes while the WE (6004) and S (6014) are shorted on the opposite electrode. The potential across the complete cell is measured. This includes contributions from the CE/electrolyte interface and the electrolyte itself. The two-electrode configuration can therefore be used whenever precise control of the interfacial potential across the WE (6004) electrochemical interface is not critical and the behavior of the whole cell is under investigation.

Three electrode setup. The three-electrode cell setup is the most common electrochemical cell setup used in electrochemistry. In this case, the current flows between the CE (6002) and the WE (6004). The potential difference is controlled between the WE (6004) and the CE (6002) and measured between the RE (6006) (preferably kept at close proximity of the WE (6004)) and S (6014). Because the WE (6004) is connected with S (6014) and WE (6004) is kept at pseudo-ground (fixed, stable potential), by controlling the polarization of the CE (6002), the potential difference between RE (6006) and WE (6004) is controlled all the time. The potential between the WE (6004) and CE (6002) usually is not measured. This is the voltage applied by the control amplifier (6008) and it is limited by the compliance voltage of the instrument. It is adjusted so that the potential difference between the WE (6004) and RE (6006) will be equal to the potential difference specified by the user. This configuration allows the potential across the electrochemical interface at the WE (6004) to be controlled with respect to the RE (6006).

Large-scale electrode arrays comprising a plurality of individually addressable electrodes formed in a circuit-supporting substrate, especially CMOS, have been constructed for phosphoramidite-based synthesis and for sensor applications, e.g. Montgomery, U.S. patents 6093302, 6444111 and 6280595; Gindilis, U.S. patent 9339782; Maurer et al, U.S. patent 9267213; Maurer et al, PLosOne, December 2006, issue 1, e34; Fomina et al, LabChip, 16: 2236-2244 (2016); Kavusi et al, U.S. patent 9075041; Johnson et al, U.S. patent 9874538 and 9910008; Gordon et al, U.S. patent 6251595; Levine et al, and the like. IEEE J. Solid State Circuits, 43: 1859-1871 (2008); and the like. These references provide guidance for the design of particular embodiments of the present invention with respect to such features as electrode numbers, size, composition and configurations at array sites; CMOS circuitry for voltage and current control and measurement; array fabrication and operation; methodologies for attaching or immobilizing chemical components (such as, for example, initiators) at array sites; and the like.

Of particular interest are the electrode configurations described in Morimoto et al, Anal. Chem. 80: 905-914 (2008); Levine et al (cited above); and Fomina et al (cited above) and their implementation with CMOS technology, particularly as described by Levine et al and Fomina et al. In some embodiments of the invention, an electrode array is provided comprising a plurality of individually addressable working electrodes in a CMOS substrate, which is operationally associated with a reference electrode and a counter electrode, the latter of which may be onboard or separate from the CMOS electrode array. CMOS circuitry is configured so that the voltage between the working electrodes and the counter electrode (s) may be adjusted to establish and maintain a desired voltage difference between selected working electrodes and the reference electrode. The desired voltage differences may be changed at selected working electrodes to cleave electrochemically labile protecting groups.

### Combination of Enzymatic Synthesis and Electrochemical Deprotection.

In one aspect, the present invention also provides a solution for combining the different ways to induced specially controlled deprotection, through pH decrease, with enzymatic DNA synthesis technology. Enzymatic synthesis is fully compatible with aqueous media. Most of the chemistry, electrochemistry or photochemistry, enabling a pH change though physical actuation are working only in aqueous media. The invention is providing technical solution to make these two aspects compatible by developing the appropriate chemistry for pH change and the appropriate buffers, reagents and protection groups for the enzymatic synthesis. So, in one of the embodiment the controllable chemistry is compatible with DNA synthesis and with the flow-cell chip surface chemistry.

Electrochemical, or Induced, deprotection, that is, the use of voltage changes at an electrode adjacent to a reaction site, has been employed to remove DMT protection groups in phosphoramidite-based synthesis, e.g. Egeland et al, Nucleic Acids Research, 33(14): e125 (2005); Montgomery (cited above). The invention in part is a discovery and recognition that parallel template-free enzymatic polynucleotide synthesis could be accomplished using electrochemical deprotection of protecting groups specific for enzymatic synthesis. In particular, 3'-O-azidomethyl protecting groups may be cleaved by direct reduction and 3'-O-amino protecting groups may be cleaved indirectly by adjusting local pH by way of an electroactive intermediary compound. For example, in the case of the latter, in some embodiments, a typical deprotection solution is 700 mM sodium nitrite (NaNO2) and 1 M sodium acetate titrated to pH 5.0-5.5 with HCl. Local deprotection of 3'-O-NH2 groups at a reaction site of an array may be effected by lowering the local pH from pH 7 to pH 5.

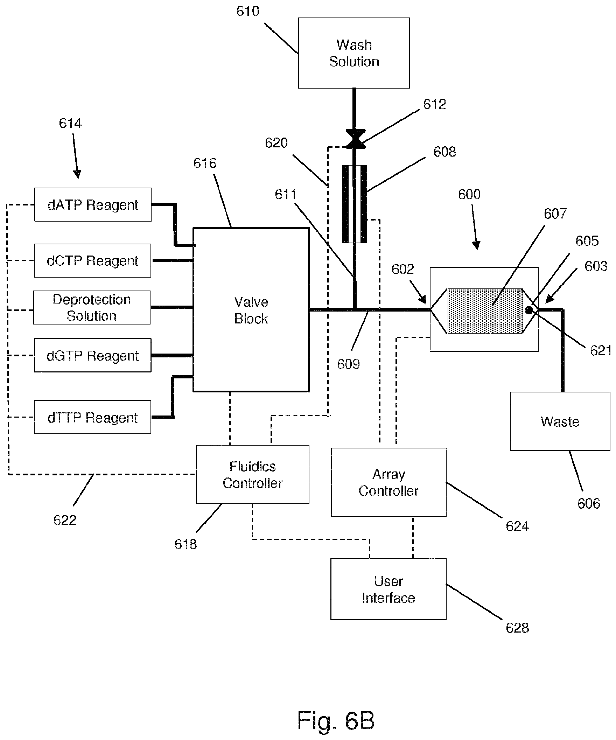

Apparatus for implementing methods of the invention. Components of an apparatus for implementing a method of the invention are illustrated diagrammatically in Fig. 6B. Flow cell and electrode array (600) comprise an array of reaction sites each of which may include a microwell, coatings to enhance attachment of initiators or other components and each of which is operationally associated with one or more electrodes. In some embodiments, the electrode array is integrated with CMOS control and measurement circuitry as a single chip. A flow cell can have a variety of designs for controlling the path and flow rate of reagents over the electrode array. In some embodiments, a flow cell is a microfluidics device. That is, it may be fabricated with micromachining techniques or precision molding to include additional fluidic passages, chambers, and so on. In one aspect, a flow cell comprises an inlet (602), an outlet (603), and a flow chamber (605) for defining the flow path of reagents over electrode array (607). Reagents are discarded into a waste container (606) after exiting flow cell and sensor array (600). In accordance with this embodiment, a function of the apparatus is to deliver different reagents to flow cell and electrode array (600) in a predetermined sequence, for predetermined durations, at predetermined flow rates, and optionally to measure physical and/or chemical parameters at the electrode sites that provide information about the status of a reaction taking place therein. To this end, fluidics controller (618) controls by lines (620 and 622) the driving forces for a plurality of reagents (614) (for example, 3'-O-protected dNTPs and/or template-free polymerase in appropriate buffers and deprotection solution(s)) and the operation of valves (for example, 612 and 616) by conventional instrument control software, e.g. Lab View (National Instruments, Austin, Tex.).

The reagents may be driven through the fluid pathways, valves and flow cell by pumps, by gas pressure, or other conventional methods. In some embodiments, a single reference electrode (608) may be positioned upstream of flow cell and sensor array (600). In other embodiments, a reference electrode may be positioned within the flow chamber. In some embodiments, a single fluid or reagent is in contact with reference electrode (608) throughout an entire multi-step reaction. This may be achieved with the configuration illustrated in Fig. 6B where reagents (614) are directed through passage (609) to flow cell (605). When those reagents are flowing, valve (612) is shut, thereby preventing any wash solution from flowing into passage (609). Although the flow of wash solution is stopped, there is still uninterrupted fluid and electrical communication between reference electrode, passage (609), and electrode array (607). At most reagents (614) when flowing through passage (609) diffuse into passage (611), but the distance between reference electrode (608) and the junction between passages (609) and (611) is selected so that little or no amount of the reagents flowing in common passage (609) reach reference electrode (608). Although Fig. 6B and other figures illustrate an electrode (for example, reference electrode, 608) as a cylinder concentric with a fluid passage (for example, 611), reference electrodes, such as (608), may have a variety of different shapes. For example, it could be a wire inserted into the lumen of (611). In one aspect, reference electrode (608) constitutes a section of passage (612) that is made of a conductive material, such as stainless steel, gold, or the like. In some embodiments, the material is inert with respect to reagents in contact with it. Reference electrode (608) in one embodiment is a tube made of a conductive material which forms part of passage (612).

The potential of the reference voltage depends on the interface between the electrode and the solution in which the electrode is in contact. For example, solutions of different nucleoside triphosphates may cause the reference voltage to change, thereby causing undesirable changes at the working electrodes. For multi-step reactions using frequent wash steps, wash solution (610) may be selected as the reagent in continuous contact with reference electrode (608) as illustrated in Fig. 6B.

Further components of this embodiment include array controller (624) for providing bias voltages (such as to control the potential between working electrodes and counter electrodes(621), which may or may not be integral with array (607)) and timing and control signals to the electrode array (if such components are not integrated into the electrode array), and for collecting and/or processing output signals. Information from flow cell and electrode array (600), as well as instrument settings and controls may be displayed and entered through user interface (628). For some embodiments, for example, nucleic acid synthesis and/or sequencing, the temperature of flow cell and sensor array (600) is controlled so that reactions take place and measurements are made at a known, and preferably, predetermined temperatures. Such temperature may be controlled by conventional temperature control devices, such as, a Peltier device, or the like. In one aspect, temperature is conveniently controlled by controlling the temperatures of the reagents flowing through the flow cell. Flow cells and fluidic circuits of the apparatus may be fabricated by a variety of methods and materials. Factors to be considered in selecting materials include degree of chemical inertness required, operating conditions, e.g. temperature, and the like, volume of reagents to be delivered, whether or not a reference voltage is required, manufacturability, and the like. For small scale fluid deliveries, microfluidic fabrication techniques are well-suited for making fluidics circuits of the invention, and guidance for such techniques is readily available to one of ordinary skill in the art, e.g. Malloy, Plastic Part Design for Injection Molding: An Introduction (Hanser Gardner Publications, 1994); Herold et al, Editors, Lab-on-a-Chip Technology (Vol. 1): Fabrication and Microfluidics (Caister Academic Press, 2009); and the like. For meso-scale and larger scale fluid deliveries, conventional machining techniques may be used to fabricate parts that may be assembled into flow cells or fluidic circuits of the invention. In one aspect, plastics such as polycarbonate, polymethyl methacrylate, and the like, may be used to fabricate flow cells and fluidics circuits of the invention.

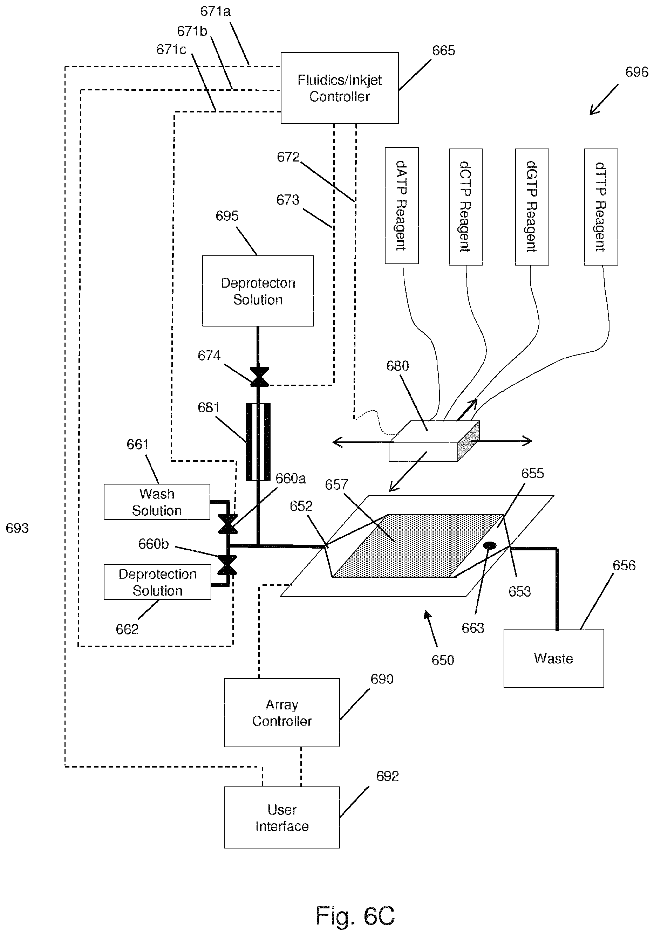

Fig. 6C illustrates diagrammatically an alternative apparatus for implementing methods of the invention wherein some reagents are delivered to reaction sites using an inkjet droplet generator. Many of the design features described above are applicable to this embodiment. As above, flow cell and electrode array (650) comprise an array of reaction sites each of which may include a microwell, coatings to enhance attachment of initiators or other components and each of which is operationally associated with one or more electrodes. As above, in some embodiments, the electrode array may be integrated with CMOS control and measurement circuitry as a single chip. A flow cell can have a variety of designs for controlling the path and flow rate of reagents over the electrode array; however, unlike the apparatus of Fig. 6B, here reaction sites of the electrode array must be accessible for delivery of reagent-containing droplets by print head (680). In one aspect, a flow cell comprises an inlet (652), an outlet (653), and a flow chamber (655) for defining the flow path of reagents (not delivered by print head (680)) over electrode array (657). Reagents are discarded into a waste container (656) after exiting flow cell and sensor array (650). In accordance with this embodiment, a function of the apparatus is to deliver different reagents either via inlet (652) or print head (680) to flow cell and electrode array (650) in a predetermined sequence, for predetermined durations, at predetermined flow rates, and optionally to measure physical and/or chemical parameters at the electrode or reaction sites that provide information about the status of a reaction taking place therein. To this end, fluidics controller (658) controls by lines (671a, 6711b and 671c) valves (660a and 660b) and print head (680). Valves (660a) and (660b) control the delivery of wash solution (661) and deprotection solution (662) (if required) to flow cell (657). Guidance for design and control of inkjet delivery systems is well known by those with skill in the art and may be found in U.S. patent publication US2003/0170698 and U.S. patents 6306599; 6323043; 7276336; 7534561; and like references.

In some embodiments, a single reference electrode (608) may be positioned upstream of flow cell and sensor array (600). In other embodiments, a reference electrode may be positioned within the flow chamber. In some embodiments, a single counter electrode (663) may be employed, or in other embodiments, more than one counter electrodes may be employed, and as described, above such counter electrodes may or may not be integrated on the same electronic substrate as the working electrodes of array (657).
The apparatus is controlled through user interface (692) which, in turn, actuates and monitors synthesis steps through fluidics/inkjet controller (665) and array controller (690) as indicated by dashed lines (671, 672 and 673). In particular, physical parameters, such as temperature, and circuitry for electrode selection, voltage control, sensor readouts, and the like, are handled by array controller (690); selection of reagents (696), droplet rates, head movement, and the like, is controlled by fluidics/inkjet controller (665). In some embodiments, during droplet delivery of 3'-O-protected dNTP monomers and/or template-free polymerase, the electrolyte connect between reaction sites and reference electrode (681) is broken as flow cell (655) may be drained to prevent cross contamination between adjacent reaction sites that receive different monomers. In some embodiments, such cross contamination may be avoided by providing reaction sites surrounded by hydrophobic regions so that each site is encompassed by an isolated liquid droplet when an electrolyte, such as, a wash solution or a deprotection solution, recedes from the flow chamber, e.g. as described in Brennan, U.S. patents 5474796, 6921636, and the like. In particular, when the flow chamber is flooded with deprotection solution a continuous electrolyte path is restored to reference electrode (681) and counter electrode(s) which may be either on array (657) or off-array.

In some implementations, the value of the voltage difference between working electrodes and reference electrode is selected to avoid unwanted redox reactions, such as electrolysis of water, so that bubbles do not form in the fluidics of the device. In some embodiments, predetermined voltage differences to bring about electrochemical reactions in the invention are about 1.5 volts or less.

In some embodiments, methods of the invention, such as implemented by the apparatus of Figs. 6B and 6C, may comprise the steps of (a) providing a spatially addressable array of reaction sites, wherein each reaction site is operationally associated with at least one working electrode and has disposed thereon initiators attached by their 5'-ends and having a 3'-O-electrochemically labile protecting group; (b) performing for each kind of nucleotide a cycle comprising steps of (i) deprotecting initiators or elongated fragments at electrodes at predetermined addresses by generating a voltage difference between each of the electrodes at the predetermined addresses and a reference electrode so that the electrochemically labile protecting group is cleaved, thereby generating free 3'-hydroxyls on the initiators or elongated fragments at the electrodes of the predetermined addresses, and (ii) contacting under elongation conditions the electrodes at the predetermined addresses with a 3'-O-electrochemically labile-protected nucleoside triphosphate and a template-independent DNA polymerase so that the initiators or elongated fragments at the predetermined addresses are elongated by the incorporation of a 3'-electrochemically labile-protected nucleoside triphosphate to form 3'-O-electrochemically labile-protected elongated fragments; and (c) repeating step (b) until the array of polynucleotides of predetermined sequences is completed. Reaction sites are generally discrete regions on a substrate within which a single kind of polynucleotide with a predetermined sequence is synthesized. Reaction sites are spatially addressable in the sense that they have well defined locations on a substrate or surface, which usually form a regular pattern, such as a rectilinear pattern, hexagonal pattern, or the like. Each reaction site is operationally associated with at least one working electrode in the sense that the electrical potential, or voltage, at the reaction site may be controlled or determined by its associated one or more working electrodes. Typically reaction sites and working electrodes are spatially aligned. That is, if an electrode is a disc or other planar structure embedded on a substrate surface, the area occupied by a reaction site corresponds to the area of the electrode surface. This is advantageous for ensuring a uniform electrical effect in reactions taking place at the reaction site. In some embodiments, a reaction site may comprise a substrate or film on a surface of a working electrode, for example, such substrate or film may be used to facilitate attachment and/or retention of components, such as, initiators. Such substrates and electrodes may be integrated in a semiconductor device, such as a CMOS device. In reference to step (b), performing the indicated cycle of steps for each kind of nucleotide is not intended to be limited to the four nucleotides A, C, G and T. In some embodiments, each kind of nucleotide means a subset of A, C, G and T. In other embodiments, each kind of nucleotide means an extended set that may include non-natural nucleotides or other nucleotide analogs that may be useful for encoding information in polynucleotides. A variety of template-independent DNA polymerases may be employed in methods of the invention; in particular, variants of terminal deoxynucleotidyl transferase are employed, e.g. as described in Ybert et al, International patent publication WO/2019/030149, or the like. The cycles of step (b) may include further steps, such as washing steps. Elongation conditions comprise buffers, salts, temperature, co-factors and the like, that are necessary or useful for incorporation activity of the template-free polymerase employed.

In some embodiments, an electrochemically labile protecting group may be pH sensitive and pH may be regulated by voltage difference between working electrodes and a reference electrode which voltage activates an electroactive agent which, in turn, changes the pH, e.g. Southern, U.S. patent 5667667; Mauer et al, U.S. patent 9267213; and the like, which are hereby incorporated by reference. Exemplary, electroactive agents include hydroquinone, benzoquinone, quinone, and derivatives thereof.

Electrochemically labile protecting groups may themselves be redox sensitive such that a voltage difference between a working electrode and a reference electrode converts the electrochemically labile protecting group into a reduced state thereby cleaving said electrochemically labile protecting group.

The apparatus described above, or like apparatus, may be used to store and retrieve information encoded in the synthesized polynucleotides. Information encoded in the polynucleotides may be retrieved by sequencing the polynucleotides. Virtually any nucleic acid sequencing technique may be used, but for some embodiments, particularly those in which sequencing take place on an electrode array, sequencing-by-synthesis techniques are of primary interest, for example, as disclosed in Bentley et al, Nature, 456: 53-59 (2008); Rothberg et al, Nature, 475: 348-352 (2011); Ravi et al, Methods Mol Biol. 1706: 223-232 (2018); and the like. In some embodiments, polynucleotides on an electrode array of the invention are sequenced or read using a sequence-by-synthesis approach that employs reversible terminators, such as, reversible terminators carrying cleavable fluorescent labels. Such sequencing methods are described in the following references, which are incorporated herein by reference: Wu et al, Proc. Natl. Acad. Sci., 104(42): 16462-16467 (2007); Guo et al, Acc. Chem. Res. 43(4): 551-563 (2010); Ju et al, Proc. Natl. Acad. Sci., 103(52): 19635-19640 (2006); Guo et al, Proc. Natl. Acad. Sci., 105(27): 9145-9150 (2008); Barnes et al, U.S. patent 7057026; and the like.

In some embodiments, methods for storing information in polynucleotides and retrieving information from such polynucleotides may comprise the following steps: (a) providing a spatially addressable array of reaction sites, wherein each reaction site is operationally associated with at least one working electrode and has disposed thereon initiators attached by their 5'-ends and having a 3'-O-electrochemically labile protecting group; (b) performing for each kind of nucleotide a cycle of (i) deprotecting initiators or elongated fragments at electrodes at predetermined addresses by generating a predetermined voltage difference between each of the electrodes at the predetermined addresses and a reference electrode so that the electrochemically labile protecting group is cleaved, thereby generating free 3'-hydroxyls on the initiators or elongated fragments at the electrodes at the predetermined addresses, (ii) contacting under elongation conditions the electrodes at the predetermined addresses with a 3'-O-electrochemically labile-protected nucleoside triphosphate and a template-independent DNA polymerase so that the initiators or elongated fragments at the predetermined addresses are elongated by the incorporation of a 3'-electrochemically labile-protected nucleoside triphosphate to form 3'-O-electrochemically labile-protected elongated fragments; (c) repeating step (b) until the array of polynucleotides of predetermined sequences is completed, wherein each of the completed polynucleotides comprises in a 5' to 3' direction an information encoding region and a sequencing primer binding site at its 3' end; and (d) retrieving information from the information encoding region by annealing a sequencing primer to the sequencing primer binding site and sequencing by synthesis the information encoding region of the completed polynucleotides at one or more reaction sites. In some embodiments, the information encoding region may contain other features, such as, additional primer binding sites, restriction sites, or the like, for processing or manipulating the polynucleotides.

In some embodiments, sequencing by synthesis may comprise incorporating a labeled reversibly blocked nucleoside triphosphate into said sequencing primer or an extension thereof by a template-dependent polymerase such that the identity of the incorporated labeled reversibly blocked nucleoside triphosphate is determined by said sequence of the polynucleotide at the reaction site. In some embodiments, the label and the blocking group of the labeled reversibly blocked nucleoside triphosphate may be attached to separate moieties of the labeled reversibly blocked nucleoside triphosphate, so that de-blocking and label removal may be accomplished by the same step or by different steps. Of particular interest are labeled reversibly blocked nucleoside triphosphate that comprise a 3'-O-electrochemically labile blocking group that is removed from the extended sequencing primers at reaction sites of predetermined addresses by generating a predetermined voltage difference between each of the electrodes at the predetermined addresses and a reference electrode. In this manner, polynucleotides at all or a predetermined subset of reaction sites may be sequenced, or read.

It is understood that the voltage differences employed to cleave the different protection or blocking groups may be different, so that reference to "a predetermined voltage difference" means a predetermined voltage difference specific for bringing about a specific effect, such as, a desired local pH change via an electroactive agent to bring about cleavage of a specific group, or direct cleavage of a specific group by its reduction, or the like.

In some embodiments it may be advantageous to synthesize polynucleotides in low quantities at discrete reaction sites then amplify them to further populate, or fill in, the reaction sites. This may be accomplished by techniques depending on thermal cycling, such as bridge PCR, or it may be accomplished by isothermal techniques, such as template walking of recombinase-polymerase amplification.

In some embodiments, either a portion of reaction sites on an array may have initiators with orthogonal 3'-O-electrochemically labile protection groups to the protection groups on initiators of the other reaction sites, or a portion of initiators within the same reaction site may comprise orthogonal 3'-O-electrochemically labile protection groups with respect to the other initiators at the same reaction site. By "orthogonal" in reference to two or more protection groups it is meant that conditions used to cleave one protection group will not affect the other protection groups, and vice versa for the conditions of removal for each protection group.

In some embodiments, at least two completed polynucleotides at different reaction sites have sequencing primer binding sites comprising different sequences, so that the at least two completed polynucleotides can be sequenced separately. In some cases, sequencing primer binding sites are attached by template-free enzymatic synthesis, in other embodiments, such primer binding sites may be ligated to synthesized polynucleotides.

In some embodiments, the different sequences of the sequencing primer binding sites are associated with different information encoded in their corresponding information encoding regions. Such different sequencing primer binding sites can index subsets of information stored in the polynucleotides of an array.

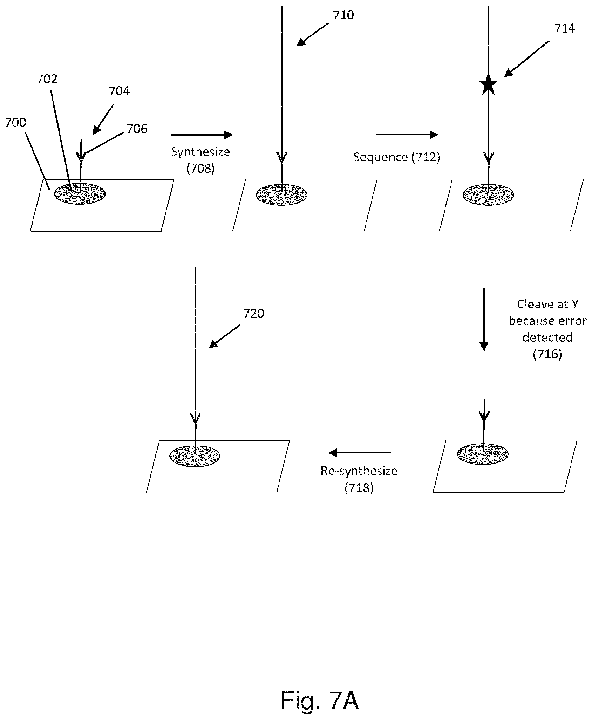

Figs. 7A-7D describe various embodiments of the invention for producing, reducing error in, storing and retrieving information from, polynucleotides whose nucleotide sequences encode information. Fig. 7A illustrates an embodiment that includes error correction. Electrode array (700) is shown with single reaction site (702) associated with an electrode (not shown) and initiator sequence (704) having a free 3'-hydroxyl and a labile bond or nucleotide (706) that is capable of being cleaved to leave a free 3'-hydroxyl. In some embodiments, bond or nucleotide (706) is electrochemically labile such that it may be selectively cleaved at reaction site (702). Initiator (704) is elongated (708) by template-free enzymatic synthesis using 3'-O-electrochemically labile-protected nucleoside triphosphate and a template-independent DNA polymerase to produce full length polynucleotide (710), after which full length polynucleotide (710) is sequenced to confirm that it has the desired sequence. If error (714) is determined, then polynucleotide (710) may be selectively cleaved at labile bond Y (706) and a replacement polynucleotide (720) may be re-synthesized (718).

In some embodiments, steps of synthesis may be monitored by detecting labeled pyrophosphate groups that are released from the dNTPs during incorporation, e.g. Fuller et al, U.S. patent 7223541, so that (for example) a profile of fluorescent signals from a reaction site will indicate whether the intended nucleotide was incorporated into the growing polynucleotide and the extent of mis-incorporation, if any.

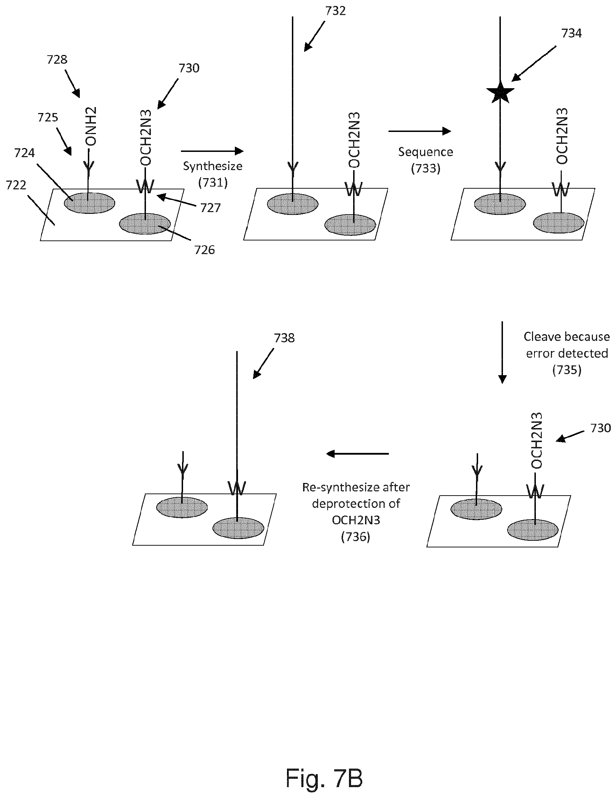

Fig. 7B illustrates another embodiment of the invention in which different reaction sites of an electrode array have initiators with orthogonal protection groups. In one application of this embodiment, polynucleotides may be synthesized on only a portion of the reaction sites by deprotecting only first protection groups on initiators at a portion of reaction sites, wherein second protection groups at the other reaction site are not removed. In alternative embodiments, the initiators with orthogonal protection groups may populate the same reaction site. As shown in Fig. 7B, electrode array (722) has reaction sites (724) and (726) each with initiators (725) and (727), respectively. Initiators (725) and (727) optionally include labile bonds "Y" and "W", respectively, and electrochemically labile protection groups, 3'-O-NH2 (728) and 3'-O-CH2N3 (730), respectively. One of the protection groups, for example, 3'-O-NH2, may be removed and polynucleotide (732) synthesized (731). If upon sequencing (733) polynucleotide (732) error (734) is discovered, then the polynucleotide with the incorrect sequence optionally may be cleaved (735), second protection group (730) at a different reaction site may be deprotected and a polynucleotide with the intended sequence may be re-synthesized (736) to produce correct sequence polynucleotide (738). Alternatively, polynucleotide (732) with error (734) may be left intact on array (722), that is, not cleaved, and its address or position annotated as containing an incorrect sequence. The error-correction schemes of Figs. 7A-7B may be used with encoding schemes that rely on redundancy or they may be used to reduce the amount of redundancy required to ensure a predetermined level of reliability of stored information.

Embodiments of Figs. 7A and 7B may be implemented with the following steps: (a) providing a spatially addressable array of reaction sites, wherein each reaction site is operationally associated with at least one working electrode and has disposed thereon initiators attached by their 5'-ends and having a 3'-O-electrochemically labile protecting group; (b) performing for each kind of nucleotide a cycle of (i) deprotecting initiators or elongated fragments at electrodes at predetermined addresses by generating a predetermined voltage difference between each of the electrodes at the predetermined addresses and a reference electrode so that the electrochemically labile protecting group is cleaved, thereby generating free 3'-hydroxyls on the initiators or elongated fragments at the electrodes at the predetermined addresses, (ii) contacting under elongation conditions the electrodes at the predetermined addresses with a 3'-O-electrochemically labile-protected nucleoside triphosphate and a template-independent DNA polymerase so that the initiators or elongated fragments at the predetermined addresses are elongated by the incorporation of a 3'-electrochemically labile-protected nucleoside triphosphate to form 3'-O-electrochemically labile-protected elongated fragments; (c) repeating step (b) until the array of polynucleotides of predetermined sequences is completed, wherein each of the completed polynucleotides comprises in a 5' to 3' direction an information encoding region and a sequencing primer binding site at its 3' end; and (d) retrieving information from the information encoding region by annealing a sequencing primer to the sequencing primer binding site and sequencing by synthesis the completed polynucleotides at one or more reaction sites. In some embodiments, each initiator comprises a cleavable nucleotide or cleavable bond such that an initiator can be cleaved at the cleavable nucleotide or cleavable bond whenever said retrieved information indicates a synthesis error. After such cleavage the polynucleotide may be re-synthesized from the cleaved initiator. In some embodiments, such cleavable bonds or nucleotides are electrochemically labile. In other embodiments, a portion of the reaction sites of an array comprise initiators with orthogonal 3'-O-eletrochemically labile protection groups with respect to other 3'-O-electrochemically labile protection groups of initiators of other reaction sites on the array. In such embodiments, methods further includes the steps of deprotecting initiators at at least one reaction site having initiators with orthogonal 3'-O-electrochemically labile protection groups and re-synthesizing the polynucleotide of the predetermined sequence whenever said retrieved information indicates a synthesis error. That is, whenever sequencing indicates an error, a polynucleotide with the desired correct sequence is re-synthesized on one of reaction sites of the portion of reaction site containing initiators with orthogonal protection groups. The polynucleotide with the incorrect sequence may be cleaved or it may be noted as having an incorrect sequence, but otherwise not cleaved from the array.

In some embodiments, synthesized polynucleotides are stored on the electrode arrays; that is, after synthesis the polynucleotides remain on the array and are stored along with the array. Information encoded in the polynucleotides may be retrieved by either sequencing the polynucleotides in situ while they remain on the array or they may be cleaved from the array and sequenced.

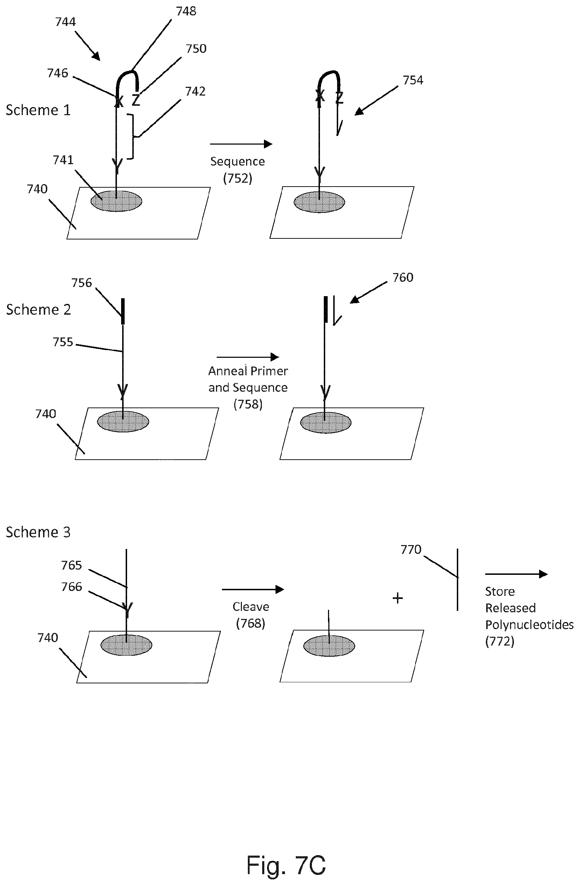

Fig. 7C illustrates three schemes for reading, or sequencing, polynucleotides on electrode array (740) with reaction sites (741). In scheme 1, after information encoding section (742) is synthesized, a self-complementary hairpin-forming section (744) is synthesized comprising sequence X (746), loop sequence (748), and sequence Z (750), the complement of sequence X (746). Under annealing conditions, Z (750) hybridizes to (746) so that sequencing reactions (752) can be performed to produce extended strand (754). As mentioned above, the resulting double stranded polynucleotide may be stored as is, or it may be cleaved from array (740) for separate storage. In some embodiments, the resulting double stranded polynucleotide may in a retrieval process be cleaved at hairpin (744) in order to separate complementary strand (754) produced in the sequencing reaction, for example, if the sequencing reaction leaves undesirable adducts. Scheme 1 has the advantage that the lack of readily accessible free 3' or 5' hydroxyl makes the resultant double stranded polynucleotide more resistant to nuclease digestion. Scheme 1 has the advantages of (i) not requiring a sequencing primer, and (ii) separate reading at any reaction site is possible if site-specific primers are selected or if electrochemically labile block groups are used in the sequencing chemistry. Scheme 2 is similar to scheme 1 except instead of hairpin (744), polynucleotide (755) is appended with primer binding site (756). Primer (760) is annealed to primer binding site (756) and a sequencing reaction is performed to read the sequence of polynucleotide (755). Scheme 2 has the advantage that repeat reads are possible on-array. Also, separate reading at any reaction site is possible if site-specific primers are selected or if electrochemically labile block groups are used in the sequencing chemistry.

In scheme 3, after synthesis of polynucleotide (765) on initiator containing cleavable bond (766), polynucleotide (765) is cleaved (768) to release strand (770) which may be stored separately from array (740).

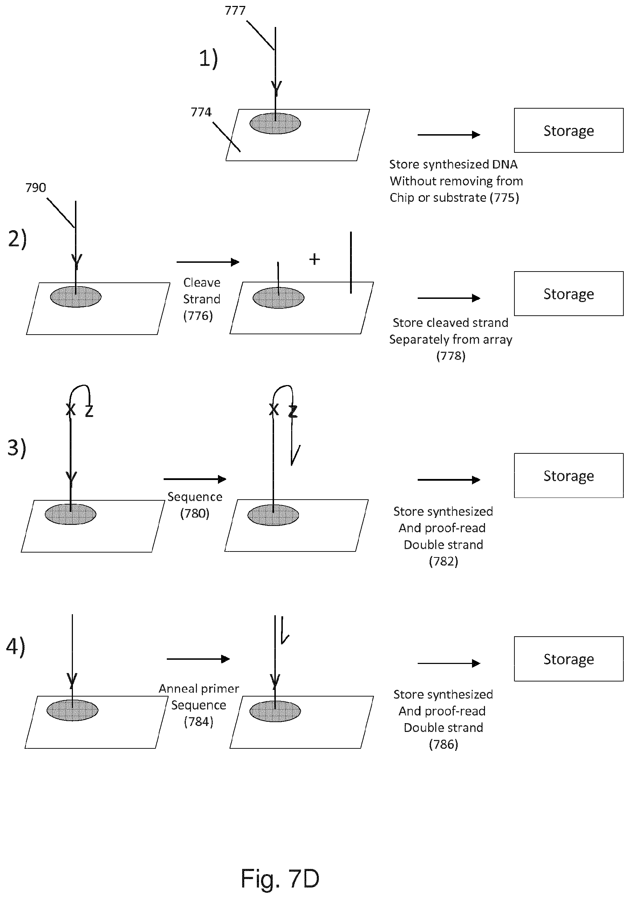

Fig. 7D reviews configurations for polynucleotide storage. Panel 1) shows the simplest storage configuration; namely, after polynucleotides (777) is synthesized on array (774), both the array and polynucleotide are stored together without cleavage of polynucleotide (777). The configuration of panel 2) is similar to that of panel 1) except that after synthesis polynucleotide (790) is cleaved (776) and stored without the array. The configurations of panels 3) and 4) correspond to synthesis schemes 1 and 2, respectively. Both configurations result in double stranded polynucleotides which have the advantage of permitting the strands to be stored separately for increased security of the encoded information.

### C. Other Synthesis Considerations.

Error Correction Process. The biggest source of errors anticipated is depurination. This degradation of DNA bases in acidic conditions leaves an abasic site in the DNA strand. When sequenced, they appear as a mix of 4 bases with SBS and are difficult to handle. A glycosylase enzyme can be used to cleave the oligonucleotides at those abasic sites, making them undetectable to sequencing (no sequencing primer) so they won't pollute the data.

Reset synthesis. If fragments above 500nt are synthesized, the use of SBS may be insufficient for their sequencing. It is thus an object of the present invention to provide a new method of sequencing long fragments, and particularly fragments above 500nt. To this end, the invention proposes to add one or several intermediary sequencing primers within the oligonucleotide to enable sequencing successive shorter oligonucleotides.

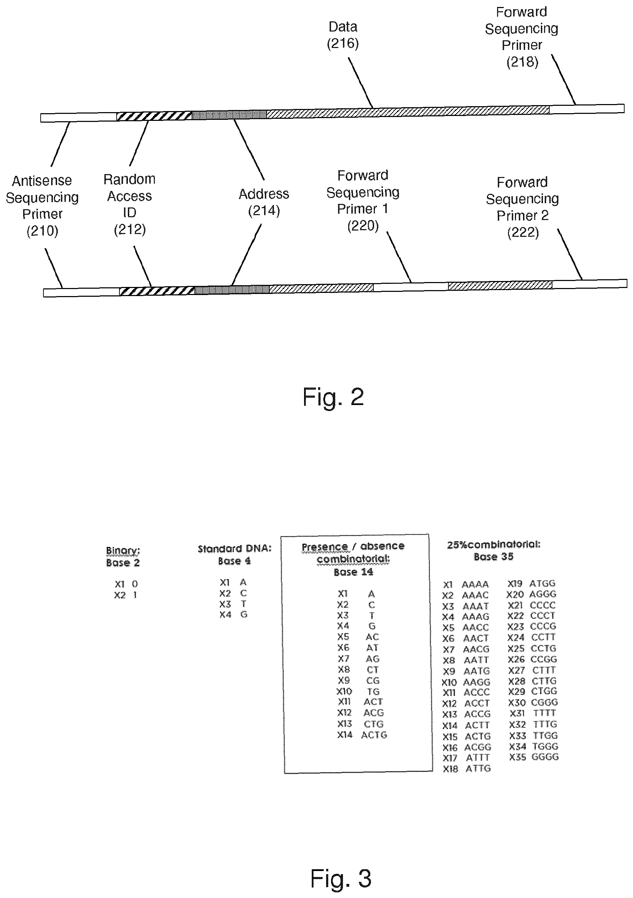

As illustrated in Figure 2, the "Data" of the oligonucleotide fragment can be fragmented into several sub-data fragments ("Data 1" and "Data 2" in Figure 2). When sequenced, this oligonucleotide can be sequenced first using Antisense sequencing primer and Forward sequencing primer 1 with dual paried ends (300nt read - Data 1, Random access ID, address), and then using Forward sequencing primer 2 (150nt - Data 2). This can be used with several intermediary forward primers to read any sequence needed. To synthesize those oligonucleotides on chip, it is possible to either synthesize the intermediary primers one nucleotide by one nucleotide or use a ligation enzyme. The error pattern after the intermediary primer is exactly the same as before.

To enable reset synthesis, the present invention also provides specific dUTP-ONH2 reversible terminator nucleotides or rUTP-ONH2 reversible terminator nucleotides. The present invention also provides enzymes variants able to incorporate at acceptable yield those 2 nucleotides (see for instance WO2017/216472). The dUTP-ONH2 may be cleaved via the action of a USER enzyme mix: uracil DNA glycosylase and endonuclease VIII during 15min; and the rUTP-ONH2 via the action of KOH 1M for 2 hours. As a result, it is possible to incorporate those nucleotides at any position in the sequence of the oligonucleotide and particularly between the data sections and the sequencing primer sections and release the different part of the data to create individual oligonucleotides from one single synthesis site.

Oligo pool creation. After synthesis, it is possible to get the library synthesized in the form of an oligo pool. There are two possibilities to get this pool:
- Cleave the oligonucleotides
- Perform an isothermal amplification on chip
The second solution gives the possibility to keep the DNA material on chip to ease sequencing.

As described below, the oligonucleotides may have sequencing primers on their 5' and 3' end. Isothermal amplification of this library thanks to these primers is envisioned. Isothermal amplification is preferred to standard PCR because cycling temperature can be damaging for the chip.

### D. Nucleic Array Synthesis Compatible With Sequencing Workflow

In a another aspect, the invention provides new nucleic array synthesis that is compatible with sequencing workflow.

It is very tedious to synthesize DNA and to sequence it afterward because the chemistry used for reading and writing are not compatible. Synthesis and sequencing have never been performed in the same instrument because they involve two very different technologies (organic chemistry for synthesis and enzymatic reactions for sequencing). For instance, phosphoramidite reagents used for phosphoramidite chemistry must be manipulated in an anhydrous media (very low traces of water will prevent the reaction from happening efficiently) whereas sequencing is performed in aqueous solutions in the case of Sequencing by synthesis, pyrosequencing, nanopore sequencing and ion semiconductor sequencing among others. For the industry this is a challenge because there is a need to verify the sequence of DNA synthesized before use. It is especially true when DNA/RNA is synthesized on chips (microarray industry or for synthesis of oligo pools).

On chip synthesis compatible with sequencing. The present invention provides a solution to the above problem by allowing on chip synthesis compatible with sequencing. According to the present invention, the DNA/RNA synthesis is performed enzymatically on flow-cells compatible with sequencing instruments. Because sequencing relies on enzymatic synthesis of a strand of DNA complementary to the strand to sequence in pyrosequencing, sequencing by synthesis and ion semiconductor sequencing, the flow cells are optimized for enzymatic synthesis, with the following aspects:
1. Possibility to inject aqueous reagents
2. Laminar flows in the chip
3. Passivation of surface to prevent DNA, enzyme and nucleotide sticking
4. Stable for multiple enzymatic cycles
DNA synthesis can be performed for instance using electrochemistry, inkjet printing or photoinduced deprotection. After synthesis, the product obtained can be array with a wide variety of oligonucleotides on its surface, each spot containing a different oligonucleotide. This configuration is exactly the one used for sequencing by synthesis. Each spot is a sequencing cluster. Adding a sequencing primer by synthesis or ligation on the 3' is the only preparation required before sequencing. Usually, sample preparation is way more complex, with several steps (dilution, ligation, PCR, bridge amplification, etc.). It can also be an array with microbeads into microwells, each microbead having oligonucleotides of a defined sequence on their surface. The instrument could even be the same for synthesis and sequencing. Photochemical synthesis and sequencing by synthesis for example could be performed in a similar instrument as they both rely on optics and need a transparent flow cell. Electrochemical synthesis and ion semiconductor (Ion Torrent) sequencing for instance also are particularly compatible.

### E. Combinatorial encoding scheme for DNA data storage.

In a further aspect, the present invention provides encoding scheme for DNA data storage. DNA data storage has the potential to disrupt the data storage market thanks to very high data density, easy and long-term storage. It thus requires very high DNA synthesis and sequencing throughput to be viable. DNA synthesis is usually performed on 2D microarray. Digital data is usually stored in base 2, and DNA is in base 4 (2 bits could theoretically be encoded in one nucleotide). One solution to increase data density (and incidentally synthesis and sequencing throughput) is to increase this encoding base further than 4. One solution has been to add unnatural DNA bases (for instance Steven Benner's AEGIS nucleotides) but it is limited and it can make sequencing harder. The present invention now proposes to increase the encoding base using only the four natural bases. This scheme could also be implemented with additional unnatural nucleotides.

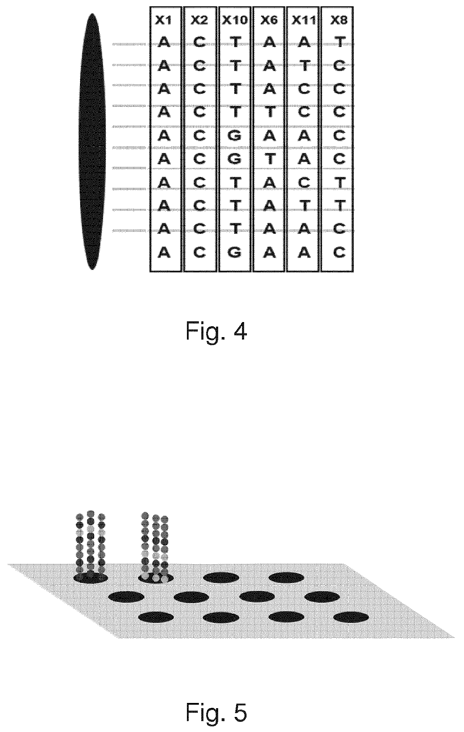

Combinatorial schemes. To further implement data density, the invention proposes is to implement a combinatorial scheme by adding mixes of nucleotides at each cycle instead of only one of the four nucleotides (Figure 3 and 4). On one well, adding a mix of nucleotide per cycle can increase the base in which the data are encoding. Using the presence/absence mechanism described above, the encoding base is 14 and it is possible to increase the data density by approximately 2. Using the 25% combinatorial scheme (Figure 3), the base is 35 enabling the encoding of 5 bits per cycle (35 > 32 = 2^5). A 10% combinatorial scheme with 10 levels (X1= AAAAAAAAAA, X2=AAAAAAAAAT, etc) would give a 268 base. This kind of combinatorial encoding can be rather easily sequenced using SBS technology and quantifying the quantity of nucleotide on each spot.

| **Encoding method** | **Number of bits per cycle** |
|---|---|
| Quaternary | 2 |
| Presence/absence combinatorial | Ln(14)/ln(2) = 3.81 |
| 25% combinatorial | Ln(35)/ln(2)=5.13 |
| 10% combinatorial | Ln(268)/ln(2)=8.07 |

Pseudo 3D data storage. This scheme can be used to increase data density when the synthesis support is stored (to be sequenced in the future if data needs to be retrieved). Keeping the synthesis support dramatically reduces the number of reads required to decipher a defined amount of data in sequencing. Indeed, when data is stored in DNA oligo pools, each fragment needs to be read an average of 50 times (read depth of 50) to be sure that every oligonucleotide has been sequenced. When the solid support is kept, the read depth is 1 as oligonucleotides are perfectly ordered. In the context of the present invention, this is named pseudo 3D data storage (Flow cell is 2D and the sequence of nucleotide represents the 3^{rd} dimension) to enable the use of DNA data storage for medium cold data storage and enable the easy readout of the digital data (Figure 5). With 1µm² wells, 400mers, and 25% combinatorial scheme, the data density would be more than 2kbit per µm² on our flow cells. This data density compares with the densest data storage media today:
- Magnetic tape: 200 GB /in² (Sony world record in 2017) = 2480 bits/µm²
- HD Bluray disc: 13.6GB /in² (4 layers blu ray) = 215 bits/µm²
- 1µm², 400mers, 25% combinatorial: >2000 bits/µm²
Using 10% combinatorial scheme would enable to achieve more than 3.5kbits/µm² which is out of reach today. If synthesis is performed in 1µm beads instead of chips, one may have more than 100TB of data in 1cm^3 which exceeds current storage technologies by far. Beads could be redeposited in sequencing chip for readout with read depth of 1.

The synthesis and sequencing methods of the invention may be used with virtually any coding scheme, such as those disclosed in the following references, which are incorporated by reference: Bornholt et al, IEEE Micro, 37(3): 98-104 (2017); Goldman et al, Nature, 494: 77-80 (2013); Chen et al, U.S. patent publication US2018/0265921; Chen et al, US20180230509A1; Strauss et al, US20170141793A1; Blawat et al, EP3067809A1; and the like.

### Cleavable Linkages and Nucleotides

A wide variety of cleavable linkages, or more particularly, cleavable nucleotides, may be used with embodiments of the invention. As used herein, the term "cleavable site" refers to a nucleotide or backbone linkage of a single stranded nucleic acid sequence that can be excised or cleaved under predetermined conditions, thereby separating the single stranded nucleic acid sequence into two parts. In some embodiments, a step of cleaving a cleavable nucleotide, a cleavable linkage or cleavable bond leaves a free 3'-hydroxyl on a cleaved strand, thereby, for example permitting the cleaved strand to be extended by a polymerase. Cleaving steps may be carried out chemically, thermally, enzymatically or by light-based cleavage. In some embodiments, cleavable nucleotides may be nucleotide analogs such as deoxyuridine or 8-oxo-deoxyguanosine that are recognized by specific glycosylases (e.g. uracil deoxyglycosylase followed by endonuclease VIII, and 8-oxoguanine DNA glycosylase, respectively). In some embodiments, cleavage by glycosylases and/or endonucleases may require a double stranded DNA substrate.

In some embodiments, cleavable nucleotides include nucleotides comprising base analogs cleavable by endonuclease III which include, but are not limited to, urea, thymine glycol, methyl tartonyl urea, alloxan, uracil glycol, 6-hydroxy-5,6-dihydrocytosine, 5-hydroxyhydantoin, 5-hydroxycytocine, trans-1-carbamoyl-2-oxo-4,5-dihydrooxyimidazolidine, 5,6-dihydrouracil, 5-hydroxycytosine, 5-hydroxyuracil, 5-hydroxy-6-hydrouracil, 5-hydroxy-6-hydrothymine, 5,6-dihydrothymine. In some embodiments, cleavable nucleotides include nucleotides comprising base analogs cleavable by formamidopyrimidine DNA glycosylase which include, but are not limited to, 7,8-dihydro-8-oxoguanine, 7,8-dihydro-8-oxoinosine, 7,8-dihydro-8-oxoadenine, 7,8-dihydro-8-oxonebularine, 4,6-diamino-5-formamidopyrimidine, 2,6-diamino-4-hydroxy-5-formamidopyrimidine, 2,6-diamino-4-hydroxy-5-N-methylformamidopyrimidine, 5-hydroxycytosine, 5-hydroxyuracil. In some embodiments, cleavable nucleotides include nucleotides comprising base analogs cleavable by hNeil 1 which include, but are not limited to, guanidinohydantoin, spiroiminodihydantoin, 5-hydroxyuracil, thymine glycol. In some embodiments, cleavable nucleotides include nucleotides comprising base analogs cleavable by thymine DNA glycosylase which include, but are not limited to, 5-formylcytosine and 5-carboxycytosine. In some embodiments, cleavable nucleotides include nucleotides comprising base analogs cleavable by human alkyladenine DNA glycosylase which include, but are not limited to, 3-methyladenine, 3-methylguanine, 7-methylguanine, 7-(2-chloroehyl)-guanine, 7-(2-hydroxyethyl)-guanine, 7-(2-ethoxyethyl)-guanine, 1,2-bis-(7-guanyl)ethane, 1,N⁶-ethenoadenine, 1,N²-ethenoguanine, N²,3-ethenoguanine, N²,3-ethanoguanine, 5-formyluracil, 5-hydroxymethyluracil, hypoxanthine. In some embodiments, cleavable nucleotides include 5-methylcytosine cleavable by 5-methylcytosine DNA glycosylase.

Exemplary chemically cleavable internucleotide linkages for use in the methods described herein include, for example, -cyano ether, 5'-deoxy-5'-aminocarbamate, 3'deoxy-3'-aminocarbamate, urea, 2'cyano-3',5'-phosphodiester, 3'-(S)-phosphorothioate, 5'-(S)-phosphorothioate, 3'-(N)-phosphoramidate, 5'-(N)-phosphoramidate, -amino amide, vicinal diol, ribonucleoside insertion, 2'-amino-3',5'-phosphodiester, allylic sulfoxide, ester, silyl ether, dithioacetal, 5'-thio-furmal, -hydroxymethyl-phosphonic bisamide, acetal, 3'-thio-furmal, methylphosphonate and phosphotriester. Internucleoside silyl groups such as trialkylsilyl ether and dialkoxysilane are cleaved by treatment with fluoride ion. Base-cleavable sites include -cyano ether, 5'-deoxy-5'-aminocarbamate, 3'-deoxy-3'-aminocarbamate, urea, 2'-cyano-3',5'-phosphodiester, 2'-amino-3',5'-phosphodiester, ester and ribose. Thio-containing internucleotide bonds such as 3'-(S)-phosphorothioate and 5'-(S)-phosphorothioate are cleaved by treatment with silver nitrate or mercuric chloride. Acid cleavable sites include 3'-(N)-phosphoramidate, 5'-(N)-phosphoramidate, dithioacetal, acetal and phosphonic bisamide. An - aminoamide internucleotide bond is cleavable by treatment with isothiocyanate, and titanium may be used to cleave a 2'-amino-3',5'-phosphodiester-O-ortho-benzyl internucleotide bond. Vicinal diol linkages are cleavable by treatment with periodate. Thermally cleavable groups include allylic sulfoxide and cyclohexene while photo-labile linkages include nitrobenzylether and thymidine dimer. Methods synthesizing and cleaving nucleic acids containing chemically cleavable, thermally cleavable, and photo-labile groups are described for example, in U.S. Pat. No. 5,700,642.

Further cleavable linkages are disclosed in the following references: Pon, R., Methods Mol. Biol. 20:465-496 (1993); Verma et al., Ann. Rev. Biochem. 67:99-134 (1998); U.S. Pat. Nos. 5,739,386, 5,700,642 and 5,830,655; and U.S. Patent Publication Nos. 2003/0186226 and 2004/0106728, Urdea et al, U.S. patent 5367066.

The cleavable site may be located along the oligonucleotide backbone, for example, a modified 3'-5' internucleotide linkage in place of one of the phosphodiester groups, such as ribose, dialkoxysilane, phosphorothioate, and phosphoramidate internucleotide linkage. The cleavable oligonucleotide analogs may also include a substituent on, or replacement of, one of the bases or sugars, such as 7-deazaguanosine, 5-methylcytosine, inosine, uridine, and the like.

Synthesis and cleavage conditions of chemically cleavable oligonucleotides are described in U.S. Pat. Nos. 5,700,642 and 5,830,655. Phosphorothioate internucleotide linkage may be selectively cleaved under mild oxidative conditions. Selective cleavage of the phosphoramidate bond may be carried out under mild acid conditions, such as 80% acetic acid. Selective cleavage of ribose may be carried out by treatment with dilute ammonium hydroxide. In another embodiment, a cleavable linking moiety may be an amino linker. The resulting oligonucleotides bound to the linker via a phosphoramidite linkage may be cleaved with 80% acetic acid yielding a 3'-phosphorylated oligonucleotide, which may (if desired) be removed by a phosphatase.

In some embodiments, the cleavable linking moiety may be a photocleavable linker, such as an ortho-nitrobenzyl photocleavable linker. Synthesis and cleavage conditions of photolabile oligonucleotides on solid supports are described, for example, in Venkatesan et al., J. Org. Chem. 61:525-529 (1996), Kahl et al., J. Org. Chem. 64:507-510 (1999), Kahl et al., J. Org. Chem. 63:4870-4871 (1998), Greenberg et al., J. Org. Chem. 59:746-753 (1994), Holmes et al., J. Org. Chem. 62:2370-2380 (1997), and U.S. Pat. No. 5,739,386. Ortho-nitrobenzyl-based linkers, such as hydroxymethyl, hydroxyethyl, and Fmoc-aminoethyl carboxylic acid linkers, may also be obtained commercially.

In some embodiments, ribonucleotides may be employed as cleavable nucleotides, wherein a cleavage step may be implemented using a ribonuclease, such as RNase H. In other embodiments, cleavage steps may be carried out by treatment with a nickase.

### Example 1

### Electrochemical Reduction of 3'-O-Azidomethyl-Nucleotide (comparative example)

In this example, conditions for the reduction of 3'-O-azidomethylnucleotides are determined by applying different voltages across electrodes in microwells for different lengths of time. The treated nucleotides were analyzed by LCMS and gel electrophoresis to determine reaction products.

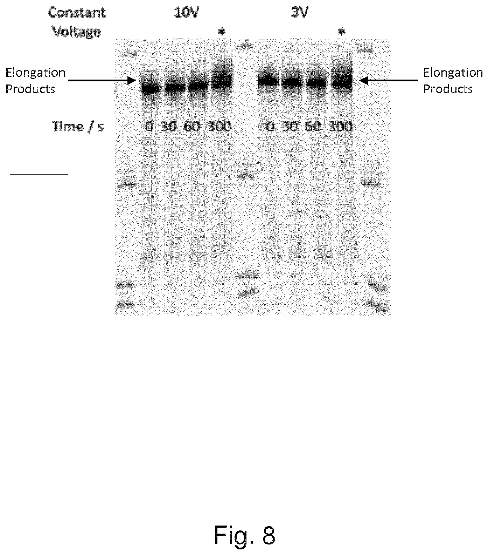

Two platinum electrodes were used to apply current to 20 uL aqueous samples (@ 9 mM) of 3' -O-azidomethyldeoxythymidine under 3 and 10 volts for different amounts of time (0, 30, 60 and 300 seconds). Evidence of 3'OH (deprotected) nucleotides in the samples was then assessed with LCMS and gel electrophoresis. Treated nucleotides were used for solution elongation of a primer by a mutant terminal deoxynucleotidyl transferase (SEQ ID NO: 1) that can couple 3'-hydroxyl nucleotides but not 3'-O-azidomethylnucleotides to the primer. The elongation reaction was as follows: 4 uM TdT, 136 uM dTTP (the treated nucleotides from microplate), 10 nmol primer (5'-FAM-polyTdU-3'-OH) with a reaction volume of 103 uL. After incubation for 10 min at 37°C and removal of unincorporated monomers (1200 rpm), the products were separated by gel electrophoresis. LCMS showed evidence of deprotection by the appearance of a 3'OH dNTP band (not shown). The electropherogram of the separated products are shown in Fig. 8. The larger bands, clearly visible for the 300 sec samples (columns indicated by *), show that deprotected dTTP was in the treated samples and the azidomethyl group had been removed electrochemically, thereby permitting extension of the primers to form n+1 products.

### Example 2

### Electrochemical Control of 3'-O-Amino-Nucleotide Deprotection

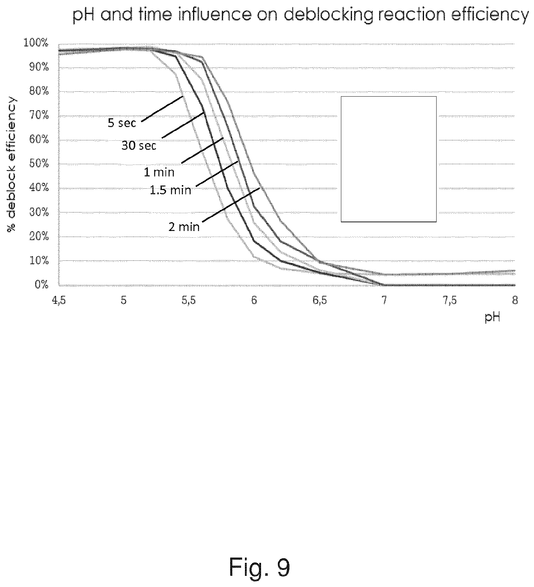

An aminoxy reversible protection group on a DNA's 3' end is cleaved by nitrosium ions (NO+) in acidic conditions, e.g. Benner, U.S. patent 7544794. In this experiment, pH of a deprotection buffer is electrochemically controlled via a quinone/hydroquinone redox system (e.g. Southern et al, U.S. patent publication US2004/0238369) to reduce locally pH from an initial value of 7.5 (yielding >0.5% deprotection after 2 minutes) to a final value of 5 (yielding >99% in less than 20 seconds). Results are illustrated in Fig. 9 where pH versus deprotection efficiency for several incubation times.

### Definitions

"Microfluidics device" means an integrated system of one or more chambers, ports, and channels that are interconnected and in fluid communication and designed for carrying out an analytical reaction or process, either alone or in cooperation with an appliance or instrument that provides support functions, such as sample introduction, fluid and/or reagent driving means, temperature control, detection systems, data collection and/or integration systems, and the like. Microfluidics devices may further include valves, pumps, and specialized functional coatings on interior walls, e.g. to prevent adsorption of sample components or reactants, facilitate reagent movement by electroosmosis, or the like. Such devices are usually fabricated in or as a solid substrate, which may be glass, plastic, or other solid polymeric materials, and typically have a planar format for case of detecting and monitoring sample and reagent movement, especially via optical or electrochemical methods. Features of a microfluidic device usually have cross-sectional dimensions of less than a few hundred square micrometers and passages typically have capillary dimensions, e.g. having maximal cross-sectional dimensions of from about 500 µm to about 0.1 µm. Microfluidics devices typically have volume capacities in the range of from 1 µL to a few nL, e.g. 10-100 nL. The fabrication and operation of microfluidics devices are well-known in the art as exemplified by the following references that are incorporated by reference: Ramsey, U.S. Pat. Nos. 6,001,229; 5,858,195; 6,010,607; and 6,033,546; Soane et al, U.S. Pat. Nos. 5,126,022 and 6,054,034; Nelson et al, U.S. Pat. 6,613,525; Maher et al, U.S. Pat. No. 6,399,952; Ricco et al, International patent publication WO 02/24322; Bjornson et al, International patent publication WO 99/19717; Wilding et al, U.S. Pat. Nos. 5,587,128; 5,498,392; Sia et al, Electrophoresis, 24: 3563-3576 (2003); Unger et al, Science, 288: 113-116 (2000); Enzelberger et al, U.S. Pat. No. 6,960,437.

"Polynucleotide" or "oligonucleotide" are used interchangeably and each mean a linear polymer of nucleotide monomers or analogs thereof. Monomers making up polynucleotides and oligonucleotides are capable of specifically binding to a natural polynucleotide by way of a regular pattern of monomer-to-monomer interactions, such as Watson-Crick type of base pairing, base stacking, Hoogsteen or reverse Hoogsteen types of base pairing, or the like. Such monomers and their internucleosidic linkages may be naturally occurring or may be analogs thereof, e.g. naturally occurring or non-naturally occurring analogs. Non-naturally occurring analogs may include PNAs, phosphorothioate internucleosidic linkages, bases containing linking groups permitting the attachment of labels, such as fluorophores, or haptens, and the like. Whenever the use of an oligonucleotide or polynucleotide requires enzymatic processing, such as extension by a polymerase, ligation by a ligase, or the like, one of ordinary skill would understand that oligonucleotides or polynucleotides in those instances would not contain certain analogs of internucleosidic linkages, sugar moieties, or bases at any or some positions. Polynucleotides typically range in size from a few monomeric units, e.g. 5-40, when they are usually referred to as "oligonucleotides," to several thousand monomeric units. Whenever a polynucleotide or oligonucleotide is represented by a sequence of letters (upper or lower case), such as "ATGCCTG," it will be understood that the nucleotides are in 5'→3' order from left to right and that "A" denotes deoxyadenosine, "C" denotes deoxycytidine, "G" denotes deoxyguanosine, and "T" denotes thymidine, "I" denotes deoxyinosine, "U" denotes uridine, unless otherwise indicated or obvious from context. Unless otherwise noted the terminology and atom numbering conventions will follow those disclosed in Strachan and Read, Human Molecular Genetics 2 (Wiley-Liss, New York, 1999). Usually polynucleotides comprise the four natural nucleosides (e.g. deoxyadenosine, deoxycytidine, deoxyguanosine, deoxythymidine for DNA or their ribose counterparts for RNA) linked by phosphodiester linkages; however, they may also comprise non-natural nucleotide analogs, e.g. including modified bases, sugars, or internucleosidic linkages. It is clear to those skilled in the art that where an enzyme has specific oligonucleotide or polynucleotide substrate requirements for activity, e.g. single stranded DNA, RNA/DNA duplex, or the like, then selection of appropriate composition for the oligonucleotide or polynucleotide substrates is well within the knowledge of one of ordinary skill, especially with guidance from treatises, such as Sambrook et al, Molecular Cloning, Second Edition (Cold Spring Harbor Laboratory, New York, 1989), and like references. Likewise, the oligonucleotide and polynucleotide may refer to either a single stranded form or a double stranded form (i.e. duplexes of an oligonucleotide or polynucleotide and its respective complement). It will be clear to one of ordinary skill which form or whether both forms are intended from the context of the terms usage.

"Primer" means an oligonucleotide, either natural or synthetic that is capable, upon forming a duplex with a polynucleotide template, of acting as a point of initiation of nucleic acid synthesis and being extended from its 3' end along the template so that an extended duplex is formed. Extension of a primer is usually carried out with a nucleic acid polymerase, such as a DNA or RNA polymerase. The sequence of nucleotides added in the extension process is determined by the sequence of the template polynucleotide. Usually primers are extended by a DNA polymerase. Primers usually have a length in the range of from 14 to 40 nucleotides, or in the range of from 18 to 36 nucleotides. Primers are employed in a variety of nucleic amplification reactions, for example, linear amplification reactions using a single primer, or polymerase chain reactions, employing two or more primers. Guidance for selecting the lengths and sequences of primers for particular applications is well known to those of ordinary skill in the art, as evidenced by the following references that are incorporated by reference: Dieffenbach, editor, PCR Primer: A Laboratory Manual, 2nd Edition (Cold Spring Harbor Press, New York, 2003).

"Sequence determination", "sequencing" or "determining a nucleotide sequence" in reference to polynucleotides includes determination of partial as well as full sequence information of the polynucleotide. That is, the terms include sequences of subsets of the full set of four natural nucleotides, A, C, G and T, such as, for example, a sequence of just A's and C's of a target polynucleotide. That is, the terms include the determination of the identities, ordering, and locations of one, two, three or all of the four types of nucleotides within a target polynucleotide. In some embodiments, the terms include the determination of the identities, ordering, and locations of two, three or all of the four types of nucleotides within a target polynucleotide. In some embodiments sequence determination may be accomplished by identifying the ordering and locations of a single type of nucleotide, e.g. cytosines, within the target polynucleotide "catcgc ... " so that its sequence is represented as a binary code, e.g. "100101 ... " representing "c-(not c)(not c)c-(not c)-c ... " and the like. In some embodiments, the terms may also include subsequences of a target polynucleotide that serve as a fingerprint for the target polynucleotide; that is, subsequences that uniquely identify a target polynucleotide within a set of polynucleotides, e.g. all different RNA sequences expressed by a cell.

## Claims

1. A method of synthesizing a plurality of polynucleotides having predetermined sequences, the method comprising the steps of:
(a) providing a spatially addressable array of reaction sites, wherein each reaction site is operationally associated with at least one working electrode and has disposed thereon initiators attached by their 5'-ends and having a 3'-O-electrochemically labile protecting group;
(b) performing for each kind of nucleotide a cycle of (i) deprotecting initiators or elongated fragments at electrodes at predetermined addresses by generating a voltage difference between each of the electrodes at the predetermined addresses and a reference electrode so that the electrochemically labile protecting group is cleaved, thereby generating free 3'-hydroxyls on the initiators or elongated fragments at the electrodes of the predetermined addresses, (ii) contacting under elongation conditions the electrodes at the predetermined addresses with a 3'-O-electrochemically labile-protected nucleoside triphosphate and a template-independent DNA polymerase so that the initiators or elongated fragments at the predetermined addresses are elongated by the incorporation of a 3'-electrochemically labile-protected nucleoside triphosphate to form 3'-O-electrochemically labile-protected elongated fragments; and
(c) repeating step (b) until the array of polynucleotides of predetermined sequences is completed,
wherein said electrochemically labile protecting group is pH sensitive and wherein said voltage difference between said electrodes at said predetermined addresses and a reference electrode activates an electroactive agent at said predetermined addresses which changes the pH at said predetermined addresses, thereby cleaving said electrochemically labile protecting group,
wherein said electrochemically labile protecting group is an amino group and wherein said spatially addressable array of reaction sites is integrated in a semiconductor device.

2. The method of claim 1, wherein said semiconductor device is a Complementary Metal Oxide Semiconductor (CMOS) device.

3. A method of synthesizing a plurality of polynucleotides having predetermined sequences, the method comprising the steps of:
(a) providing a spatially addressable array of reaction sites, wherein each reaction site is operationally associated with at least one working electrode and has disposed thereon initiators attached by their 5'-ends and having a 3'-O-electrochemically labile protecting group;
(b) performing for each kind of nucleotide a cycle of (i) deprotecting initiators or elongated fragments at electrodes at predetermined addresses by generating a voltage difference between each of the electrodes at the predetermined addresses and a reference electrode so that the electrochemically labile protecting group is cleaved, thereby generating free 3'-hydroxyls on the initiators or elongated fragments at the electrodes of the predetermined addresses, (ii) contacting under elongation conditions the electrodes at the predetermined addresses with a 3'-O-electrochemically labile-protected nucleoside triphosphate and a template-independent DNA polymerase so that the initiators or elongated fragments at the predetermined addresses are elongated by the incorporation of a 3'-electrochemically labile-protected nucleoside triphosphate to form 3'-O-electrochemically labile-protected elongated fragments; and
(c) repeating step (b) until the array of polynucleotides of predetermined sequences is completed,
wherein said electrochemically labile protecting group is an amino group and wherein said voltage difference between said electrodes at said predetermined addresses and a reference electrode activates an electroactive agent at said predetermined addresses which changes the pH at said predetermined addresses, thereby cleaving said electrochemically labile protecting group, wherein said electroactive agent is selected from hydroquinone, benzoquinone, quinone, and derivatives thereof.

4. A method of storing and retrieving information in an array of polynucleotides, the method comprising the steps of:
(a) providing a spatially addressable array of reaction sites, wherein each reaction site is operationally associated with at least one working electrode and has disposed thereon initiators attached by their 5'-ends and having a 3'-O-electrochemically labile protecting group;
(b) performing for each kind of nucleotide a cycle of (i) deprotecting initiators or elongated fragments at electrodes at predetermined addresses by generating a predetermined voltage difference between each of the electrodes at the predetermined addresses and a reference electrode so that the electrochemically labile protecting group is cleaved, thereby generating free 3'-hydroxyls on the initiators or elongated fragments at the electrodes at the predetermined addresses, (ii) contacting under elongation conditions the electrodes at the predetermined addresses with a 3'-O-electrochemically labile-protected nucleoside triphosphate and a template-independent DNA polymerase so that the initiators or elongated fragments at the predetermined addresses are elongated by the incorporation of a 3'-electrochemically labile-protected nucleoside triphosphate to form 3'-O-electrochemically labile-protected elongated fragments;
(c) repeating step (b) until the array of polynucleotides of predetermined sequences is completed, wherein each of the completed polynucleotides comprises in a 5' to 3' direction an information encoding region and a sequencing primer binding site at its 3' end; and
(d) retrieving information from the information encoding region by annealing a sequencing primer to the sequencing primer binding site and sequencing by synthesis the completed polynucleotides at one or more reaction sites,
wherein said electrochemically labile protecting group is an amino group.

5. The method of claim 4, wherein said sequencing by synthesis comprises incorporating a labeled reversibly blocked nucleoside triphosphate into said sequencing primer or an extension thereof by a template-dependent polymerase such that the identity of the incorporated labeled reversibly blocked nucleoside triphosphate is determined by said sequence of said polynucleotide at said reaction site.

6. The method of claim 5, wherein said labeled reversibly blocked nucleoside triphosphate comprises a 3'-O-electrochemically labile blocking group that is removed from said extended sequencing primers at reaction sites of predetermined addresses by generating a predetermined voltage difference between each of the electrodes at the predetermined addresses and said reference electrode.

7. The method of claim 5 or 6, wherein said label of said labeled reversibly blocked nucleoside triphosphates is attached thereto by an electrochemically labile bond that may be cleaved at predetermined reaction sites by generating a predetermined voltage difference between working electrodes at each predetermined address and said reference electrode.

8. The method of any one of claims 4 to 7, wherein each of said completed polynucleotides is isothermally amplified at its reaction site, preferably said isothermal amplification being carried out by template walking or recombinase-polymerase amplification.

9. The method of any one of claims 4 to 8, wherein a portion of said initiators have orthogonal 3'-O-protecting groups with respect to said 3'-O-electrochemically labile protecting group.

10. The method of claim 9, wherein said orthogonal 3'-O-protecting groups are removed and wherein each of said completed polynucleotides is isothermally amplified at its electrode.

11. The method of any one of claims 4 to 10, wherein at least two of said completed polynucleotides at different said electrodes have said sequencing primer binding sites comprising different sequences, so that the at least two said completed polynucleotides can be sequenced separately, preferably said different sequences being associated with different information encoded in their corresponding information encoding regions.

12. The method of any one of claims 4 to 11, wherein said method of sequencing by synthesis comprises steps of de-blocking an electrochemically labile 3'-O-blocking group of an extended sequencing primer and wherein such step comprises generating a predetermined voltage difference between each of said electrodes and a reference electrode so that the electrochemically labile protecting group is cleaved and/or
wherein each of said initiators comprises a cleavable nucleotide or cleavable bond and wherein said method of claim s2 further includes the steps of cleaving said initiator at the cleavable nucleotide or cleavable bond whenever said retrieved information indicates a synthesis error and re-synthesizing said polynucleotide from the cleaved initiator, and/or
wherein a portion of said reaction sites comprise initiators with orthogonal 3'-O-eletrochemically labile protection groups to said other 3'-O-electrochemically labile protection groups of initiators of other reaction sites, and wherein said method of claim 2 further includes the steps of deprotecting initiators at least one reaction site having initiators with orthogonal 3'-O-electrochemically labile protection groups and re-synthesizing a polynucleotide of a predetermined sequence whenever said retrieved information indicates a synthesis error.

13. A method of template-free enzymatic synthesis of a polynucleotide with proofreading, the method comprising the steps of:
a) providing an initiator at a reaction site operationally associated with at least one working electrode, wherein the initiator has a free 3-O-hydroxyl;
b) repeating cycles of (i) contacting under elongation conditions the initiator or an elongated fragment thereof having free 3'-O-hydroxyls with a 3'-O-electrochemically labile-protected nucleoside triphosphate comprising a 3'-O-electrochemically labile protecting group, and a template-independent DNA polymerase so that the initiator or elongated fragment thereof is elongated by the incorporation of a 3'-electrochemically labile-protected nucleoside triphosphate to form 3'-O-electrochemically labile-protected elongated fragment; and (ii) deprotecting the elongated fragment of step (i) to form elongated fragment having a free 3'-hydroxyl, until the polynucleotide is complete and a sequencing primer binding site is appended to its 3' end;
c) annealing a sequencing primer to the sequencing primer binding site and sequencing the polynucleotide,
wherein said 3'-O-electrochemically labile protecting group is an amino group and wherein said voltage difference between said electrodes at said predetermined addresses activates an electroactive agent at said predetermined addresses which changes the pH at said predetermined addresses, thereby cleaving said 3'-O-electrochemically labile protecting group.

14. The method of claim 13, wherein said sequencing by synthesis comprises incorporating a labeled reversibly blocked nucleoside triphosphate into said sequencing primer or an extension thereof by a template-dependent polymerase such that the identity of the incorporated labeled reversibly blocked nucleoside triphosphate is determined by said sequence of said polynucleotide at said reaction site.

15. The method of claim 14 wherein said labeled reversibly blocked nucleoside triphosphate comprises a 3'-O-electrochemically labile blocking group that is removed from said extended sequencing primers at reaction sites of predetermined addresses by generating a voltage difference between each of the electrodes at the predetermined addresses and a reference electrode so that the electrochemically labile blocking group is cleaved.

16. The method of any one of claims 14 to 15, wherein said label of said labeled reversibly blocked nucleoside triphosphates is attached thereto by an electrochemically labile bond that may be cleaved at predetermined reaction sites by generating a voltage difference between working electrodes at each predetermined address and a reference electrode.

17. The method of any one of claims 13 to 16, wherein said initiator comprises a cleavable nucleotide or cleavable bond and wherein said method further includes the steps of cleaving said initiator at the cleavable nucleotide or cleavable bond whenever said step of sequencing indicates a synthesis error and re-synthesizing said polynucleotide from the cleaved initiator.

## Patentansprüche

1. Verfahren zur Synthese einer Vielzahl von Polynukleotiden mit vorbestimmten Sequenzen, wobei das Verfahren die folgenden Schritte umfasst:
• (a) Bereitstellen eines räumlich adressierbaren Arrays von Reaktionsorten, wobei jeder Reaktionsort funktionell mit mindestens einer Arbeitselektrode verknüpft ist und darauf angeordnete Initiatoren aufweist, die über ihre 5'-Enden gebunden sind und eine 3'-O-elektrochemisch labile Schutzgruppe aufweisen;
• (b) Durchführen eines Zyklus für jede Nukleotidart aus:
• (i) Entschützen von Initiatoren oder verlängerten Fragmenten an Elektroden an vorbestimmten Adressen durch Erzeugen einer Spannungsdifferenz zwischen jeder der Elektroden an den vorbestimmten Adressen und einer Referenzelektrode, so dass die elektrochemisch labile Schutzgruppe abgespalten wird, wodurch freie 3'-Hydroxyle an den Initiatoren oder verlängerten Fragmenten an den Elektroden der vorbestimmten Adressen erzeugt werden,
• (ii) Inkontaktbringen der Elektroden an den vorbestimmten Adressen unter Verlängerungsbedingungen mit einem 3'-O-elektrochemisch labil geschützten Nukleosidtriphosphat und einer matrizenunabhängigen DNA-Polymerase, so dass die Initiatoren oder verlängerten Fragmente an den vorbestimmten Adressen durch den Einbau eines 3'-elektrochemisch labil geschützten Nukleosidtriphosphats verlängert werden, um 3'-O-elektrochemisch labil geschützte verlängerte Fragmente zu bilden; und
• (c) Wiederholen von Schritt (b), bis das Array von Polynukleotiden mit vorbestimmten Sequenzen fertiggestellt ist,
wobei die elektrochemisch labile Schutzgruppe pH-empfindlich ist und wobei die Spannungsdifferenz zwischen den Elektroden an den vorbestimmten Adressen und einer Referenzelektrode ein elektroaktives Agens an den vorbestimmten Adressen aktiviert, das den pH-Wert an den vorbestimmten Adressen ändert, wodurch die elektrochemisch labile Schutzgruppe abgespalten wird,
wobei die elektrochemisch labile Schutzgruppe eine Aminogruppe ist und wobei das räumlich adressierbare Array von Reaktionsorten in ein Halbleiterbauelement integriert ist.

2. Verfahren nach Anspruch 1, wobei das Halbleiterbauelement ein CMOS-Bauelement (Complementary Metal Oxide Semiconductor) ist.

3. Verfahren zur Synthese einer Vielzahl von Polynukleotiden mit vorbestimmten Sequenzen, wobei das Verfahren die folgenden Schritte umfasst:
• (a) Bereitstellen eines räumlich adressierbaren Arrays von Reaktionsorten, wobei jeder Reaktionsort funktionell mit mindestens einer Arbeitselektrode verknüpft ist und darauf angeordnete Initiatoren aufweist, die über ihre 5'-Enden gebunden sind und eine 3'-O-elektrochemisch labile Schutzgruppe aufweisen;
• (b) Durchführen eines Zyklus für jede Nukleotidart aus:
• (i) Entschützen von Initiatoren oder verlängerten Fragmenten an Elektroden an vorbestimmten Adressen durch Erzeugen einer Spannungsdifferenz zwischen jeder der Elektroden an den vorbestimmten Adressen und einer Referenzelektrode, so dass die elektrochemisch labile Schutzgruppe abgespalten wird, wodurch freie 3'-Hydroxyle an den Initiatoren oder verlängerten Fragmenten an den Elektroden der vorbestimmten Adressen erzeugt werden,
• (ii) Inkontaktbringen der Elektroden an den vorbestimmten Adressen unter Verlängerungsbedingungen mit einem 3'-O-elektrochemisch labil geschützten Nukleosidtriphosphat und einer matrizenunabhängigen DNA-Polymerase, so dass die Initiatoren oder verlängerten Fragmente an den vorbestimmten Adressen durch den Einbau eines 3'-elektrochemisch labil geschützten Nukleosidtriphosphats verlängert werden, um 3'-O-elektrochemisch labil geschützte verlängerte Fragmente zu bilden; und
• (c) Wiederholen von Schritt (b), bis das Array von Polynukleotiden mit vorbestimmten Sequenzen fertiggestellt ist,
wobei die elektrochemisch labile Schutzgruppe eine Aminogruppe ist und wobei die Spannungsdifferenz zwischen den Elektroden an den vorbestimmten Adressen und einer Referenzelektrode ein elektroaktives Agens an den vorbestimmten Adressen aktiviert, das den pH-Wert an den vorbestimmten Adressen ändert, wodurch die elektrochemisch labile Schutzgruppe abgespalten wird, wobei das elektroaktive Agens aus Hydrochinon, Benzochinon, Chinon und Derivaten davon ausgewählt ist.

4. Verfahren zur Speicherung und zum Abrufen von Informationen in einem Array von Polynukleotiden, wobei das Verfahren die folgenden Schritte umfasst:
• (a) Bereitstellen eines räumlich adressierbaren Arrays von Reaktionsorten, wobei jeder Reaktionsort funktionell mit mindestens einer Arbeitselektrode verknüpft ist und darauf angeordnete Initiatoren aufweist, die über ihre 5'-Enden gebunden sind und eine 3'-O-elektrochemisch labile Schutzgruppe aufweisen;
• (b) Durchführen eines Zyklus für jede Nukleotidart aus:
• (i) Entschützen von Initiatoren oder verlängerten Fragmenten an Elektroden an vorbestimmten Adressen durch Erzeugen einer vorbestimmten Spannungsdifferenz zwischen jeder der Elektroden an den vorbestimmten Adressen und einer Referenzelektrode, so dass die elektrochemisch labile Schutzgruppe abgespalten wird, wodurch freie 3'-Hydroxyle an den Initiatoren oder verlängerten Fragmenten an den Elektroden an den vorbestimmten Adressen erzeugt werden,
• (ii) Inkontaktbringen der Elektroden an den vorbestimmten Adressen unter Verlängerungsbedingungen mit einem 3'-O-elektrochemisch labil geschützten Nukleosidtriphosphat und einer matrizenunabhängigen DNA-Polymerase, so dass die Initiatoren oder verlängerten Fragmente an den vorbestimmten Adressen durch den Einbau eines 3'-elektrochemisch labil geschützten Nukleosidtriphosphats verlängert werden, um 3'-O-elektrochemisch labil geschützte verlängerte Fragmente zu bilden;
• (c) Wiederholen von Schritt (b), bis das Array von Polynukleotiden mit vorbestimmten Sequenzen fertiggestellt ist, wobei jedes der fertiggestellten Polynukleotide in einer 5'-zu-3'-Richtung eine Informationskodierungsregion und eine Sequenzierungsprimer-Bindungsstelle an seinem 3'-Ende umfasst; und
• (d) Abrufen von Informationen aus der Informationskodierungsregion durch Anlagern eines Sequenzierungsprimers an die Sequenzierungsprimer-Bindungsstelle und Sequenzieren durch Synthese der fertiggestellten Polynukleotide an einem oder mehreren Reaktionsorten,
wobei die elektrochemisch labile Schutzgruppe eine Aminogruppe ist.

5. Verfahren nach Anspruch 4, wobei das Sequenzieren durch Synthese das Einbauen eines markierten, reversibel blockierten Nukleosidtriphosphats in den Sequenzierungsprimer oder eine Verlängerung davon durch eine matrizenabhängige Polymerase umfasst, so dass die Identität des eingebauten markierten, reversibel blockierten Nukleosidtriphosphats durch die Sequenz des Polynukleotids an dem Reaktionsort bestimmt wird.

6. Verfahren nach Anspruch 5, wobei das markierte, reversibel blockierte Nukleosidtriphosphat eine 3'-O-elektrochemisch labile Blockierungsgruppe umfasst, die von den verlängerten Sequenzierungsprimern an Reaktionsorten vorbestimmter Adressen durch Erzeugen einer vorbestimmten Spannungsdifferenz zwischen jeder der Elektroden an den vorbestimmten Adressen und der Referenzelektrode entfernt wird.

7. Verfahren nach Anspruch 5 oder 6, wobei die Markierung des markierten, reversibel blockierten Nukleosidtriphosphats daran über eine elektrochemisch labile Bindung gebunden ist, die an vorbestimmten Reaktionsorten durch Erzeugen einer vorbestimmten Spannungsdifferenz zwischen Arbeitselektroden an jeder vorbestimmten Adresse und der Referenzelektrode abgespalten werden kann.

8. Verfahren nach einem der Ansprüche 4 bis 7, wobei jedes der fertiggestellten Polynukleotide an seinem Reaktionsort isothermal amplifiziert wird, vorzugsweise wobei die isotherme Amplifikation durch Template-Walking oder Recombinase-Polymerase-Amplifikation durchgeführt wird.

9. Verfahren nach einem der Ansprüche 4 bis 8, wobei ein Teil der Initiatoren orthogonale 3'-O-Schutzgruppen in Bezug auf die 3'-O-elektrochemisch labile Schutzgruppe aufweist.

10. Verfahren nach Anspruch 9, wobei die orthogonalen 3'-O-Schutzgruppen entfernt werden und wobei jedes der fertiggestellten Polynukleotide an seiner Elektrode isothermal amplifiziert wird.

11. Verfahren nach einem der Ansprüche 4 bis 10, wobei mindestens zwei der fertiggestellten Polynukleotide an verschiedenen Elektroden Sequenzierungsprimer-Bindungsstellen aufweisen, die unterschiedliche Sequenzen umfassen, so dass die mindestens zwei fertiggestellten Polynukleotide getrennt sequenziert werden können, vorzugsweise wobei die unterschiedlichen Sequenzen mit unterschiedlichen Informationen verknüpft sind, die in ihren entsprechenden Informationskodierungsregionen kodiert sind.

12. Verfahren nach einem der Ansprüche 4 bis 11, wobei das Verfahren zum Sequenzieren durch Synthese Schritte des Deblockierens einer elektrochemisch labilen 3'-O-Blockierungsgruppe eines verlängerten Sequenzierungsprimers umfasst und wobei ein solcher Schritt das Erzeugen einer vorbestimmten Spannungsdifferenz zwischen jeder der Elektroden und einer Referenzelektrode umfasst, so dass die elektrochemisch labile Schutzgruppe abgespalten wird, und/oder
wobei jeder der Initiatoren ein spaltbares Nukleotid oder eine spaltbare Bindung umfasst und wobei das Verfahren nach Anspruch 2 ferner die Schritte des Spaltens des Initiators an dem spaltbaren Nukleotid oder der spaltbaren Bindung beinhaltet, wann immer die abgerufene Information einen Synthesefehler anzeigt, und des Neusynthetisierens des Polynukleotids aus dem gespaltenen Initiator, und/oder
wobei ein Teil der Reaktionsorte Initiatoren mit orthogonalen 3'-O-elektrochemisch labilen Schutzgruppen zu den anderen 3'-O-elektrochemisch labilen Schutzgruppen von Initiatoren anderer Reaktionsorte umfasst, und wobei das Verfahren nach Anspruch 2 ferner die Schritte des Entschützens von Initiatoren an mindestens einem Reaktionsort mit Initiatoren mit orthogonalen 3'-O-elektrochemisch labilen Schutzgruppen und des Neusynthetisierens eines Polynukleotids einer vorbestimmten Sequenz beinhaltet, wann immer die abgerufene Information einen Synthesefehler anzeigt.

13. Verfahren zur matrizenfreien enzymatischen Synthese eines Polynukleotids mit Korrekturlesen, wobei das Verfahren die folgenden Schritte umfasst:
• a) Bereitstellen eines Initiators an einem Reaktionsort, der funktionell mit mindestens einer Arbeitselektrode verknüpft ist, wobei der Initiator ein freies 3-O-Hydroxyl aufweist;
• b) Wiederholen von Zyklen aus:
• (i) Inkontaktbringen des Initiators oder eines verlängerten Fragments davon mit freien 3'-O-Hydroxylen unter Verlängerungsbedingungen mit einem 3'-O-elektrochemisch labil geschützten Nukleosidtriphosphat, das eine 3'-O-elektrochemisch labile Schutzgruppe umfasst, und einer matrizenunabhängigen DNA-Polymerase, so dass der Initiator oder das verlängerte Fragment davon durch den Einbau eines 3'-elektrochemisch labil geschützten Nukleosidtriphosphats verlängert wird, um ein 3'-O-elektrochemisch labil geschütztes verlängertes Fragment zu bilden; und
• (ii) Entschützen des verlängerten Fragments aus Schritt (i), um ein verlängertes Fragment mit einem freien 3'-Hydroxyl zu bilden, bis das Polynukleotide vollständig ist und eine Sequenzierungsprimer-Bindungsstelle an sein 3'-Ende angefügt ist;
• c) Anlagern eines Sequenzierungsprimers an die Sequenzierungsprimer-Bindungsstelle und Sequenzieren des Polynukleotids,
wobei die 3'-O-elektrochemisch labile Schutzgruppe eine Aminogruppe ist und wobei die Spannungsdifferenz zwischen den Elektroden an den vorbestimmten Adressen ein elektroaktives Agens an den vorbestimmten Adressen aktiviert, das den pH-Wert an den vorbestimmten Adressen ändert, wodurch die 3'-O-elektrochemisch labile Schutzgruppe abgespalten wird.

14. Verfahren nach Anspruch 13, wobei das Sequenzieren durch Synthese das Einbauen eines markierten, reversibel blockierten Nukleosidtriphosphats in den Sequenzierungsprimer oder eine Verlängerung davon durch eine matrizenabhängige Polymerase umfasst, so dass die Identität des eingebauten markierten, reversibel blockierten Nukleosidtriphosphats durch die Sequenz des Polynukleotids an dem Reaktionsort bestimmt wird.

15. Verfahren nach Anspruch 14, wobei das markierte, reversibel blockierte Nukleosidtriphosphat eine 3'-O-elektrochemisch labile Blockierungsgruppe umfasst, die von den verlängerten Sequenzierungsprimern an Reaktionsorten vorbestimmter Adressen durch Erzeugen einer Spannungsdifferenz zwischen jeder der Elektroden an den vorbestimmten Adressen und einer Referenzelektrode entfernt wird, so dass die elektrochemisch labile Blockierungsgruppe abgespalten wird.

16. Verfahren nach einem der Ansprüche 14 bis 15, wobei die Markierung des markierten, reversibel blockierten Nukleosidtriphosphats daran über eine elektrochemisch labile Bindung gebunden ist, die an vorbestimmten Reaktionsorten durch Erzeugen einer Spannungsdifferenz zwischen Arbeitselektroden an jeder vorbestimmten Adresse und einer Referenzelektrode abgespalten werden kann.

17. Verfahren nach einem der Ansprüche 13 bis 16, wobei der Initiator ein spaltbares Nukleotid oder eine spaltbare Bindung umfasst und wobei das Verfahren ferner die Schritte des Spaltens des Initiators an dem spaltbaren Nukleotid oder der spaltbaren Bindung beinhaltet, wann immer der Schritt des Sequenzierens einen Synthesefehler anzeigt, und des Neusynthetisierens des Polynukleotids aus dem gespaltenen Initiator.

## Revendications

1. Procédé de synthèse d'une pluralité de polynucléotides ayant des séquences prédéterminées, le procédé comprenant les étapes de :
• (a) fourniture d'un réseau spatialement adressable de sites de réaction, dans lequel chaque site de réaction est associé de manière opérationnelle à au moins une électrode de travail et présente, disposés sur celui-ci, des amorceurs attachés par leurs extrémités 5' et ayant un groupe protecteur 3'-O-électrochimiquement labile ;
• (b) réalisation pour chaque type de nucléotide d'un cycle de :
• (i) déprotection d'amorceurs ou de fragments allongés au niveau d'électrodes à des adresses prédéterminées en générant une différence de tension entre chacune des électrodes aux adresses prédéterminées et une électrode de référence de sorte que le groupe protecteur électrochimiquement labile soit clivé, générant ainsi des 3'-hydroxyles libres sur les amorceurs ou fragments allongés au niveau des électrodes des adresses prédéterminées,
• (ii) mise en contact, dans des conditions d'allongement, des électrodes aux adresses prédéterminées avec un nucléoside triphosphate protégé par un groupe 3'-O-électrochimiquement labile et une ADN polymérase indépendante de la matrice de sorte que les amorceurs ou fragments allongés aux adresses prédéterminées soient allongés par l'incorporation d'un nucléoside triphosphate protégé par un groupe 3'-électrochimiquement labile pour former des fragments allongés protégés par un groupe 3'-O-électrochimiquement labile ; et
• (c) répétition de l'étape (b) jusqu'à ce que le réseau de polynucléotides de séquences prédéterminées soit achevé,
dans lequel ledit groupe protecteur électrochimiquement labile est sensible au pH et dans lequel ladite différence de tension entre lesdites électrodes auxdites adresses prédéterminées et une électrode de référence active un agent électroactif auxdites adresses prédéterminées qui modifie le pH auxdites adresses prédéterminées, clivant ainsi ledit groupe protecteur électrochimiquement labile,
dans lequel ledit groupe protecteur électrochimiquement labile est un groupe amino et dans lequel ledit réseau spatialement adressable de sites de réaction est intégré dans un dispositif à semi-conducteur.

2. Procédé selon la revendication 1, dans lequel ledit dispositif à semi-conducteur est un dispositif à semi-conducteur métal-oxyde complémentaire (CMOS).

3. Procédé de synthèse d'une pluralité de polynucléotides ayant des séquences prédéterminées, le procédé comprenant les étapes de :
• (a) fourniture d'un réseau spatialement adressable de sites de réaction, dans lequel chaque site de réaction est associé de manière opérationnelle à au moins une électrode de travail et présente, disposés sur celui-ci, des amorceurs attachés par leurs extrémités 5' et ayant un groupe protecteur 3'-O-électrochimiquement labile ;
• (b) réalisation pour chaque type de nucléotide d'un cycle de :
• (i) déprotection d'amorceurs ou de fragments allongés au niveau d'électrodes à des adresses prédéterminées en générant une différence de tension entre chacune des électrodes aux adresses prédéterminées et une électrode de référence de sorte que le groupe protecteur électrochimiquement labile soit clivé, générant ainsi des 3'-hydroxyles libres sur les amorceurs ou fragments allongés au niveau des électrodes des adresses prédéterminées,
• (ii) mise en contact, dans des conditions d'allongement, des électrodes aux adresses prédéterminées avec un nucléoside triphosphate protégé par un groupe 3'-O-électrochimiquement labile et une ADN polymérase indépendante de la matrice de sorte que les amorceurs ou fragments allongés aux adresses prédéterminées soient allongés par l'incorporation d'un nucléoside triphosphate protégé par un groupe 3'-électrochimiquement labile pour former des fragments allongés protégés par un groupe 3'-O-électrochimiquement labile ; et
• (c) répétition de l'étape (b) jusqu'à ce que le réseau de polynucléotides de séquences prédéterminées soit achevé,
dans lequel ledit groupe protecteur électrochimiquement labile est un groupe amino et dans lequel ladite différence de tension entre lesdites électrodes auxdites adresses prédéterminées et une électrode de référence active un agent électroactif auxdites adresses prédéterminées qui modifie le pH auxdites adresses prédéterminées, clivant ainsi ledit groupe protecteur électrochimiquement labile, dans lequel ledit agent électroactif est choisi parmi l'hydroquinone, la benzoquinone, la quinone et des dérivés de celles-ci.

4. Procédé de stockage et de récupération d'informations dans un réseau de polynucléotides, le procédé comprenant les étapes de :
• (a) fourniture d'un réseau spatialement adressable de sites de réaction, dans lequel chaque site de réaction est associé de manière opérationnelle à au moins une électrode de travail et présente, disposés sur celui-ci, des amorceurs attachés par leurs extrémités 5' et ayant un groupe protecteur 3'-O-électrochimiquement labile ;
• (b) réalisation pour chaque type de nucléotide d'un cycle de :
• (i) déprotection d'amorceurs ou de fragments allongés au niveau d'électrodes à des adresses prédéterminées en générant une différence de tension prédéterminée entre chacune des électrodes aux adresses prédéterminées et une électrode de référence de sorte que le groupe protecteur électrochimiquement labile soit clivé, générant ainsi des 3'-hydroxyles libres sur les amorceurs ou fragments allongés au niveau des électrodes aux adresses prédéterminées,
• (ii) mise en contact, dans des conditions d'allongement, des électrodes aux adresses prédéterminées avec un nucléoside triphosphate protégé par un groupe 3'-O-électrochimiquement labile et une ADN polymérase indépendante de la matrice de sorte que les amorceurs ou fragments allongés aux adresses prédéterminées soient allongés par l'incorporation d'un nucléoside triphosphate protégé par un groupe 3'-électrochimiquement labile pour former des fragments allongés protégés par un groupe 3'-O-électrochimiquement labile ;
• (c) répétition de l'étape (b) jusqu'à ce que le réseau de polynucléotides de séquences prédéterminées soit achevé, dans lequel chacun des polynucléotides achevés comprend dans une direction 5' vers 3' une région codant de l'information et un site de liaison d'amorce de séquençage à son extrémité 3' ; et
• (d) récupération d'informations à partir de la région codant de l'information par hybridation d'une amorce de séquençage au site de liaison d'amorce de séquençage et séquençage par synthèse des polynucléotides achevés au niveau d'un ou plusieurs sites de réaction,
dans lequel ledit groupe protecteur électrochimiquement labile est un groupe amino.

5. Procédé selon la revendication 4, dans lequel ledit séquençage par synthèse comprend l'incorporation d'un nucléoside triphosphate bloqué de manière réversible et marqué dans ladite amorce de séquençage ou une extension de celle-ci par une polymérase dépendante de la matrice de sorte que l'identité du nucléoside triphosphate bloqué de manière réversible et marqué incorporé soit déterminée par ladite séquence dudit polynucléotide au niveau dudit site de réaction.

6. Procédé selon la revendication 5, dans lequel ledit nucléoside triphosphate bloqué de manière réversible et marqué comprend un groupe de blocage 3'-O-électrochimiquement labile qui est éliminé desdites amorces de séquençage étendues au niveau de sites de réaction d'adresses prédéterminées en générant une différence de tension prédéterminée entre chacune des électrodes aux adresses prédéterminées et ladite électrode de référence.

7. Procédé selon la revendication 5 ou 6, dans lequel ledit marqueur desdits nucléosides triphosphates bloqués de manière réversible et marqués y est attaché par une liaison électrochimiquement labile qui peut être clivée au niveau de sites de réaction prédéterminés en générant une différence de tension prédéterminée entre des électrodes de travail à chaque adresse prédéterminée et ladite électrode de référence.

8. Procédé selon l'une quelconque des revendications 4 à 7, dans lequel chacun desdits polynucléotides achevés est amplifié de manière isotherme au niveau de son site de réaction, de préférence ladite amplification isotherme étant effectuée par cheminement sur matrice (« template walking ») ou par amplification recombinase-polymérase.

9. Procédé selon l'une quelconque des revendications 4 à 8, dans lequel une partie desdits amorceurs présentent des groupes protecteurs en 3'-O orthogonaux par rapport audit groupe protecteur 3'-O-électrochimiquement labile.

10. Procédé selon la revendication 9, dans lequel lesdits groupes protecteurs en 3'-O orthogonaux sont éliminés et dans lequel chacun desdits polynucléotides achevés est amplifié de manière isotherme au niveau de son électrode.

11. Procédé selon l'une quelconque des revendications 4 à 10, dans lequel au moins deux desdits polynucléotides achevés au niveau de différentes électrodes susmentionnées présentent des sites de liaison d'amorce de séquençage comprenant des séquences différentes, de sorte que les au moins deux polynucléotides achevés susmentionnés puissent être séquencés séparément, de préférence lesdites différentes séquences étant associées à différentes informations codées dans leurs régions codant de l'information correspondantes.

12. Procédé selon l'une quelconque des revendications 4 à 11, dans lequel ledit procédé de séquençage par synthèse comprend des étapes de déblocage d'un groupe de blocage en 3'-O électrochimiquement labile d'une amorce de séquençage étendue et dans lequel une telle étape comprend la génération d'une différence de tension prédéterminée entre chacune desdites électrodes et une électrode de référence de sorte que le groupe protecteur électrochimiquement labile soit clivé et/ou dans lequel chacun desdits amorceurs comprend un nucléotide clivable ou une liaison clivable et dans lequel ledit procédé selon la revendication 2 comprend en outre les étapes de clivage dudit amorceur au niveau du nucléotide clivable ou de la liaison clivable chaque fois que ladite information récupérée indique une erreur de synthèse et de resynthèse dudit polynucléotide à partir de l'amorceur clivé, et/ou dans lequel une partie desdits sites de réaction comprennent des amorceurs avec des groupes de protection 3'-O-électrochimiquement labiles orthogonaux auxdits autres groupes de protection 3'-O-électrochimiquement labiles d'amorceurs d'autres sites de réaction, et dans lequel ledit procédé selon la revendication 2 comprend en outre les étapes de déprotection d'amorceurs au niveau d'au moins un site de réaction ayant des amorceurs avec des groupes de protection 3'-O-électrochimiquement labiles orthogonaux et de resynthèse d'un polynucléotide d'une séquence prédéterminée chaque fois que ladite information récupérée indique une erreur de synthèse.

13. Procédé de synthèse enzymatique sans matrice d'un polynucléotide avec relecture, le procédé comprenant les étapes de :
• a) fourniture d'un amorceur au niveau d'un site de réaction associé de manière opérationnelle à au moins une électrode de travail, dans lequel l'amorceur présente un 3-O-hydroxyle libre ;
• b) répétition de cycles de :
• (i) mise en contact, dans des conditions d'allongement, de l'amorceur ou d'un fragment allongé de celui-ci ayant des 3'-O-hydroxyles libres avec un nucléoside triphosphate protégé par un groupe 3'-O-électrochimiquement labile comprenant un groupe protecteur 3'-O-électrochimiquement labile, et une ADN polymérase indépendante de la matrice de sorte que l'amorceur ou le fragment allongé de celui-ci soit allongé par l'incorporation d'un nucléoside triphosphate protégé par un groupe 3'-électrochimiquement labile pour former un fragment allongé protégé par un groupe 3'-O-électrochimiquement labile ; et
• (ii) déprotection du fragment allongé de l'étape (i) pour former un fragment allongé ayant un 3'-hydroxyle libre, jusqu'à ce que le polynucléotide soit complet et qu'un site de liaison d'amorce de séquençage soit ajouté à son extrémité 3' ;
• c) hybridation d'une amorce de séquençage au site de liaison d'amorce de séquençage et séquençage du polynucléotide,
dans lequel ledit groupe protecteur 3'-O-électrochimiquement labile est un groupe amino et dans lequel ladite différence de tension entre lesdites électrodes auxdites adresses prédéterminées active un agent électroactif auxdites adresses prédéterminées qui modifie le pH auxdites adresses prédéterminées, clivant ainsi ledit groupe protecteur 3'-O-électrochimiquement labile.

14. Procédé selon la revendication 13, dans lequel ledit séquençage par synthèse comprend l'incorporation d'un nucléoside triphosphate bloqué de manière réversible et marqué dans ladite amorce de séquençage ou une extension de celle-ci par une polymérase dépendante de la matrice de sorte que l'identité du nucléoside triphosphate bloqué de manière réversible et marqué incorporé soit déterminée par ladite séquence dudit polynucléotide au niveau dudit site de réaction.

15. Procédé selon la revendication 14, dans lequel ledit nucléoside triphosphate bloqué de manière réversible et marqué comprend un groupe de blocage 3'-O-électrochimiquement labile qui est éliminé desdites amorces de séquençage étendues au niveau de sites de réaction d'adresses prédéterminées en générant une différence de tension entre chacune des électrodes aux adresses prédéterminées et une électrode de référence de sorte que le groupe de blocage électrochimiquement labile soit clivé.

16. Procédé selon l'une quelconque des revendications 14 à 15, dans lequel ledit marqueur desdits nucléosides triphosphates bloqués de manière réversible et marqués y est attaché par une liaison électrochimiquement labile qui peut être clivée au niveau de sites de réaction prédéterminés en générant une différence de tension entre des électrodes de travail à chaque adresse prédéterminée et une électrode de référence.

17. Procédé selon l'une quelconque des revendications 13 à 16, dans lequel ledit amorceur comprend un nucléotide clivable ou une liaison clivable et dans lequel ledit procédé comprend en outre les étapes de clivage dudit amorceur au niveau du nucléotide clivable ou de la liaison clivable chaque fois que ladite étape de séquençage indique une erreur de synthèse et de resynthèse dudit polynucléotide à partir de l'amorceur clivé.
